# EUROPEAN PATENT SPECIFICATION

(11) **EP 0 768 380 B1**
(45) Date of publication and mention of the grant of the patent: **23.08.2006**
(21) Application number: 96307177.4
(22) Date of filing: 01.10.1996
(51) Int. Cl.: C12N 15/31, C12N 15/11, C07K 14/38

(54) **Gene regulating aureobasidin sensitivity**
Ein die Aureobasidinempfindlichkeit regulierendes Gen
Gène qui règle la sensibilité à auréobasidine

(30) Priority: 04.10.1995 JP 27992195
(43) Date of publication of application: 16.04.1997
(73) Proprietor: TAKARA BIO INC., Otsu-shi, Shiga (JP)
(72) Inventor: Okado, Takashi, Soraku-gun, Kyoto-fu (JP); Takesako, Kazutoh, Otsu-shi, Shiga-ken (JP); Kato, Ikunoshin, Uhi-shi, Kyoto-fu (JP)
(74) Representative: Wakerley, Helen Rachael

(56) References cited:
- EP-A- 0 644 262
- EP-A- 0 692 534
- HEIDLER S A ET AL: "THE AUR1 GENE IN SACCHAROMYCES CEREVISIAE ENCODES DOMINANT RESISTANCE TO THE ANTIFUNGAL AGENT AUREOBASIDIN A (LY295337)" ANTIMICROBIAL AGENTS AND CHEMOTHERAPY, vol. 39, no. 12, 1 December 1995, pages 2765-2769, XP000574724
- HASHIDA-OKADO T. ET AL.: "CLONING AND CHARACTERIZATION OF A GENE CONFERRING RESISTANCE TO THE ANTIFUNGAL ANTIBIOTIC AUREOBASIDIN A(R106-I) IN YEAST" FASEB JOURNAL, vol. 9, no. 6, 24 April 1995, page A1371 XP000578257
- DICKSON R.C. ET AL.: "Synthesis of mannose-(inositol-P)2-ceramide, the majr sphingolipid on Saccharomyces cerevisiae, requires the IPT1 (YDR072c) gene" JOURNAL OF BIOLOGICAL CHEMISTRY., vol. 272, no. 47, 21 November 1997, MD US, pages 29620-29625, XP002061276
- DATABASE EMBL [Online] 13 June 1994 'R. norvegicus gene for NADH-cytochrome b5 reductase' Database accession no. RNNADHCB5
- DATABASE EMBL [Online] 24 May 1995 'Escherichia coli acrA, acrB and acrR genes' Database accession no. ECU00734
- DATABASE EMBL [Online] 27 April 1993 'Pea histone H2A mRNA' Database accession no. M64838

## Description

### [Detailed Description of the Invention]

### [Technical Field of the Invention]

This invention relates to a protein regulating a sensitivity to an antimycotic aureobasidin, and obtained from a mold such as one belonging to the genus Aspergillus and a gene encoding this protein, namely, a gene regulating aureobasidin sensitivity and originating in a mold.

### [Prior Art]

Aureobasidin [Japanese Patent Laid-Open No. 138296/1990, No. 22995/1991, No. 220199/1991, No. 279384/1993 and No. 65291/1994; J. Antibiotics, 44, (9), 919 - 924; ibid., 44, (9) 925 - 933; and ibid., 44, (11), 1187 - 1198 (1991)] is a cyclic depsipeptide obtained as a fermentation product of a strain *Aureobasidium pullulans* No. R106. It is completely different in structure from other antimycotics. Aureobasidin A, which is a typical aureobasidin compound, exerts a potent antimycotic activity on various yeasts of the genus *Candida* including *Candida albicans* (hereinafter referred to simply as *C. albicans*) which is a pathogenic fungi, *Cryptococcus neoformans, Histoplasma capsulatum, Blastomyces dermatitidis* and fungi belonging to the genera Aspergillus and Penicillium (Japanese Patent Laid-Open No. 138296/1990) but has an extremely low toxicity. Thus this compound is expected as an antimycotic being excellent in selective toxicity.

Each of the existing antimycotics with a low toxicity shows only a fungistatic action, which causes a clinical problem. In contrast, aureobasidin exerts a germicidal action. Although it has been required to clarify the mechanism of the selective toxicity of aureobasidin from these viewpoints, this mechanism still remains completely unknown.

As described in Canadian Patent Laid-Open No. 2124034, the present inventors have previously found out that Saccharomyces *cerevisiae* (hereinafter referred to simply as S. *cerevisiae)* and *Schizosaccharomyces pombe* (hereinafter referred to simply as *Schizo.* pombe) are sensitive to aureobasidin. We have further mutated sensitive cells of *S. cerevisiae* or *Schizo*. *pombe* into resistant cells and successfully isolated a gene capable of imparting a resistance to aureobasidin (a resistant gene) therefrom. We have furthermore successfully isolated a gene capable of imparting aureobasidin sensitivity (a sensitive gene) from the corresponding sensitive cells.

We have also isolated a gene regulating aureobasidin sensitivity from *C. albicans* with the use of the gene regulating aureobasidin sensitivity or a part thereof as a probe. However no gene regulating aureobasidin sensitivity has been found in molds including those belonging to the genus Aspergillus.

### [Problems to be Solved by the Invention]

There are a number of molds such as the ones of the genera Aspergillus and Penicillium. Some of these molds have been applied to food manufacturing (for example, brewing of liquors, soy sauce and miso, ripening of cheese, etc.) for a long time, while a number of them are important in the production of enzyme preparations or antibiotics. However, molds include not only these useful ones as described above but also harmful ones such as those inducing plant diseases and those causing serious human diseases such as deep-seated mycosis. The recent development in genetic engineering techniques has made it possible not only to breed useful strains but also to apply molds to novel purposes, for example, the production of a heterogenic protein. Also, analyses of vital phenomena of molds are under way.

The present invention can provide a gene, which encodes a protein regulating aureobasidin sensitivity and which is useful in genetic engineering techniques and in analyses of vital phenomena of molds, from molds including those belonging to the genus Aspergillus and its functional derivative. That is to say, the present invention aims at revealing a gene which encodes a protein regulating aureobasidin sensitivity or its functional derivative; providing a method for cloning this gene and a protein regulating aureobasidin sensitivity encoded by this gene or its functional derivative; providing the antisense DNA and the antisense RNA of this gene; providing a nucleic acid probe hybridizable with this gene and a method for detecting this gene by using this nucleic acid probe; and providing a process for producing a protein regulating aureobasidin sensitivity or its functional derivative by using this gene.

The present invention may be summarized as follows. The first aspect of the invention relates to a gene originating in a mold which encodes a protein regulating aureobasidin sensitivity or its functional derivative. Namely, it relates to a gene regulating aureobasidin sensitivity obtained from a mold or a functional derivative thereof. The second aspect of the invention relates to the antisense DNA of a gene regulating aureobasidin sensitivity represented by SEQ ID NO.13. The third aspect of the invention relates to the antisense RNA of a gene regulating aureobasidin represented by SEQ ID NO.14. The fourth aspect of the invention relates to a recombinant plasmid which contains a gene regulating aureobasidin sensitivity and originating in a mold or its functional derivative. The fifth aspect of the invention relates to a transformant which has the plasmid of the fourth aspect introduced thereinto. The sixth aspect of the invention relates to a process for producing a protein regulating aureobasidin sensitivity or its functional derivative with the use of the above-mentioned transformant. The seventh aspect of the invention relates to a protein regulating aureobasidin sensitivity and originating in a mold or its functional derivative. The eigth aspect of the invention relates to a protein capable of imparting the resistance to aureobasidin, wherein at least the amino acid Gly at the position 275 of the protein imparting aureobasidin sensitivity represented by SEQ ID NO. 4 in the Sequence Listing has been replaced by another amino acid, or its functional derivative. The ninth aspect of the invention relates to a DNA which encodes the protein of the eigth aspect capable of imparting the resistance to aureobasidin.

The present inventors have found out that molds such as *Aspergillus nidulans* (hereinafter referred to simply as *A. nidulans*) and *Aspergillus fumigatus* (hereinafter referred to simply as *A. fumigatus*) are sensitive to aureobasidin. Thus they have mutated sensitive cells of *A. nidulans* into resistant cells and succeeded in the isolation of a gene capable of imparting the resistance to aureobasidin (a resistant gene) from the corresponding resistant cells. Further, they disclose the existence of a protein encoded by this gene. They have also successfully found novel genes regulating aureobasidin sensitivity from aureobasidin sensitive *A. nidulans* and *A. fumigatus* by using a DNA fragment of the above-mentioned gene as a probe. Furthermore, they have found out that the detection of this gene enables the diagnosis of diseases caused by these cells (for example, mycosis caused by fungi) and that the antisense DNA or antisense RNA, which inhibits the expression of the gene regulating aureobasidin sensitivity characteristic of the cells, is usable as a remedy for diseases caused by these cells (for example, an antimycotic for mycosis), thus completing the present invention.

The term "a protein regulating aureobasidin sensitivity" as used herein means a protein which is contained in a mold showing a sensitivity to aureobasidin. This protein is required for achieving a sensitivity or resistance to aureobasidin. The term "a gene regulating aureobasidin sensitivity" means a gene which encodes such a protein regulating aureobasidin sensitivity and a sensitive gene and a resistant gene fall within this category. The aureobasidin sensitivity of an organism varies depending on the molecular structure or amount of such a protein or gene regulating aureobasidin sensitivity carried by the organism.

The term "a functional derivative of the protein or gene regulating aureobasidin sensitivity" as used herein means one which has a biological activity substantially comparable to that of the protein or DNA regulating aureobasidin sensitivity. It include fragments, variants, mutants, analogs, homologs and chemical derivatives. A variant means one which is substantially analogous to the whole protein or a fragment originating therein in structure and/or function. That is to say, one molecule which is essentially analogous to another in activity is regarded as a mutant, even though these two molecules are different in molecular structure or amino acid sequence from each other. The functional derivatives include proteins showing an amino acid sequence with at least one modification selected from among replacement, insertion and deletion of amino acid residue(s) and having a comparable biological activity and genes encoding these. The protein regulating aureobasidin sensitivity may be subjected to the replacement, insertion and deletion of amino acid residues by a site-specific mutagenesis. The isolated DNA encoding the protein regulating aureobasidin sensitivity can be easily subjected to at least one modification selected from among replacement, insertion and deletion of nucleotides and thus a novel DNA encoding the protein regulating aureobasidin sensitivity and its functional derivatives can be obtained.

Regarding the replacement, insertion and deletion of amino acid residues, one or more amino acids can be converted by genetic engineering techniques and those suffering from no injury to the biological activity should be selected. To properly effect a mutation on the residue at a specified site, mutagenesis is performed at random on the target codon and a mutant having the desired activity is screened from the ones thus expressed. The mutant obtained by insertion involves a fused protein wherein the protein regulating aureobasidin sensitivity or its functional derivative or a fragment thereof is bound via a peptide bond to another protein or polypeptide at the amino terminal and/or the carboxy terminal of the protein regulating aureobasidin sensitivity or its functional derivative or a fragment thereof. To delete amino acid residue(s), an arbitrary amino acid codon in the amino acid sequence may be replaced by a termination codon by the site-specific mutagenesis. Thus the region on the carboxy terminal side of the replaced amino acid residue can be deleted from the amino acid sequence. Alternatively a DNA coding for a protein, from which the amino terminal and/or carboxy terminal regions in an arbitral length have been deleted, can be obtained by the deletion method comprising degrading a coding DNA from the region(s) corresponding to the amino terminal and/or the carboxy terminal of the amino acid sequence [Gene, 33. 103 - 119 (1985)] or a PCR method with the use of primers containing an initiation codon and/or a termination codon. Known examples of the site-specific mutagenesis method include the gapped duplex method with the use of oligonucleotide(s) [Methods in Enzymology, 154, 350 - 367 (1987)], the uracil DNA method with the use of oligonucleotide(s) [Methods in Enzymology, 154, 367 382 (1987)], the nitrous acid mutation method [Proc. Natl. Acad. Sci. USA, 79, 7258 - 7262 (1982)] and the cassette mutation method [Gene, 34, 315 - 323 (1985)].

The first aspect of the invention relates to a gene regulating aureobasidin sensitivity obtained from a mold exemplified by one belonging to the genus Aspergillus or its functional derivative. In order to isolate this gene, aureobasidin sensitive cells are first subjected to a mutagenesis to thereby derive a resistant strain therefrom. Then a DNA library is prepared from the chromosome DNAs or cDNAs of this resistant strain and a gene capable of imparting the resistance (a resistant gene) is cloned from this library. Similarly, a DNA library of a sensitive strain is prepared and DNA molecules hybridizable with the resistant gene are isolated and cloned. Thus a sensitive gene can be isolated.

The mutagenesis is performed by, for example, treating with a chemical such as ethylmethane sulfonate (EMS) or N-methyl-N'-nitro-N-nitrosoguanidine (NTG) or by ultraviolet or other radiation. A mutant that has acquired the resistance can be screened by culturing the mutagenized cells in a nutritional medium containing aureobasidin at an appropriate concentration under appropriate conditions. The resistant strain thus obtained may vary depending on the method and conditions selected for the mutagenesis. It is further possible to select strains differing in the extent of resistance by varying the aureobasidin concentration at the screening. It is also possible to select a temperature-sensitive resistant strain by varying the temperature at the screening. Since there are two or more mechanisms of the resistance to aureobasidin, two or more resistant genes can be isolated by genetically classifying these resistant strains.

The genes regulating aureobasidin sensitivity of molds belonging to the genus *Aspergillus* of the present invention include a gene anaurl^{R} isolated from a resistant mutant of *A. nidulans,* a gene anaurl^{S} isolated from a sensitive strain of *A. nidulans* and a gene afaurl^{S} isolated from a sensitive strain of *A. fumigatus*.

The attached Fig. 1 shows the restriction enzyme map of the genomic DNA of the gene anaurl^{R} regulating aureobasidin sensitivity and originating in a mold of *Aspergillus,* Fig. 2 shows the restriction enzyme map of the cDNA of the gene anaurl^{s} and Fig. 3 shows the restriction enzyme map of the cDNA of the gene afaurl^{s}.

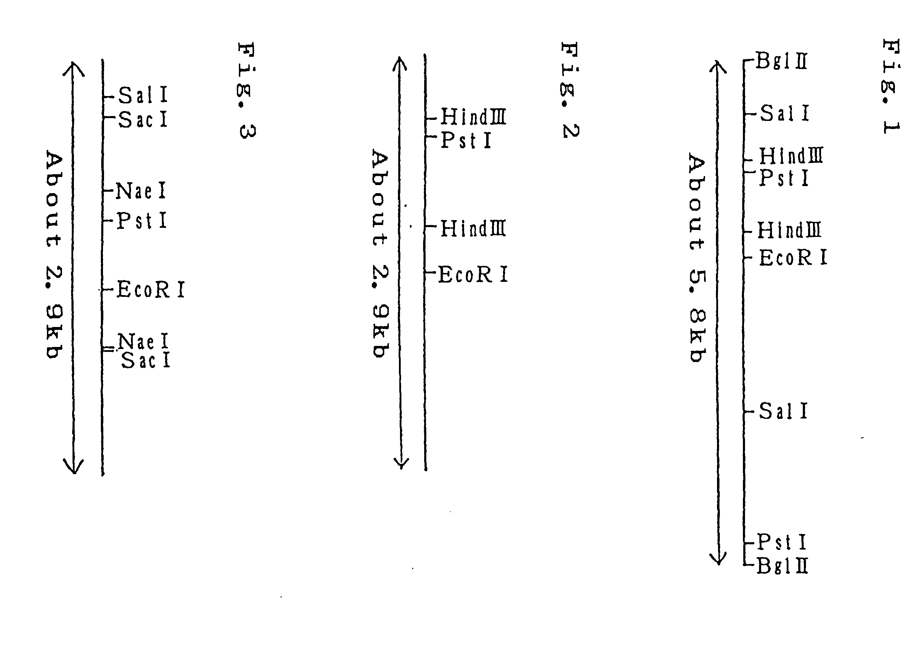

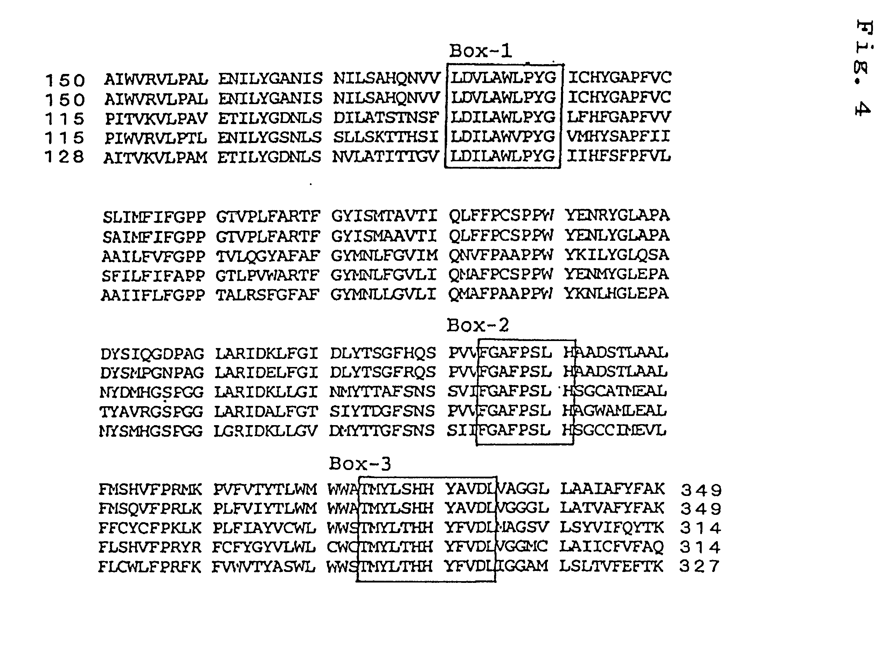

*A. nidulans* sensitive to aureobasidin is mutagenized by UV irradiation and a genomic library of the resistant strain thus obtained is prepared. From this library, a DNA fragment containing a resistant gene (anaurl^{R}) and having the restriction enzyme map of Fig. 1 is isolated. This gene has a DNA sequence represented by SEQ ID NO. 1 in the Sequence Listing. The amino acid sequence of a protein encoded by this gene, which is estimated on the basis of this DNA sequence, is the one represented by SEQ ID NO. 2 in the Sequence Listing. By the hybridization with the use of this resistant gene, a cDNA fragment containing a sensitive gene (anaurl^{s}) and having the restriction enzyme map of Fig. 2 is isolated from a cDNA library of a sensitive strain. This sensitive gene has a DNA sequence represented by SEQ ID NO. 3 in the Sequence Listing. The amino acid sequence of a protein encoded by this gene, which is estimated on the basis of this base sequence, is the one represented by SEQ ID NO. 4 in the Sequence Listing. A comparison between the sequences of SEQ ID NO. 3 and SEQ ID NO. 1 reveals that the genomic DNA has one intron (intervening sequence) ranging from the base at the position 1508 to the one at the position 1563 in SEQ ID NO. 1. Further, G at the position 1965 in SEQ ID NO. 1 has been mutated into T. A comparison between the sequences of SEQ ID NO. 4 and SEQ ID NO. 2 reveals that the amino acid glycine at the position 275 has been mutated into valine at the amino acid level, thus giving the resistance. The first aspect of the invention also involves genes constructed by chemically or physically altering a part of the genes of the present invention which regulate aureobasidin sensitivity and originate in molds.

The gene of the first aspect of the invention, its fonctional derivatives, or a part of the same can be used as or probe in a method for cloning a gene regulating aureobasidin sensitivity and originating in a mold such as one of the genus *Aspergillus* or its functional derivative.

That is to say, a gene encoding a protein having a comparable function can be isolated by the hybridization method or the polymerase chain reaction (PCR) method with the use of the whole or a part of the gene (consisting of at least 15 oligonucleotides) obtained above as a probe.

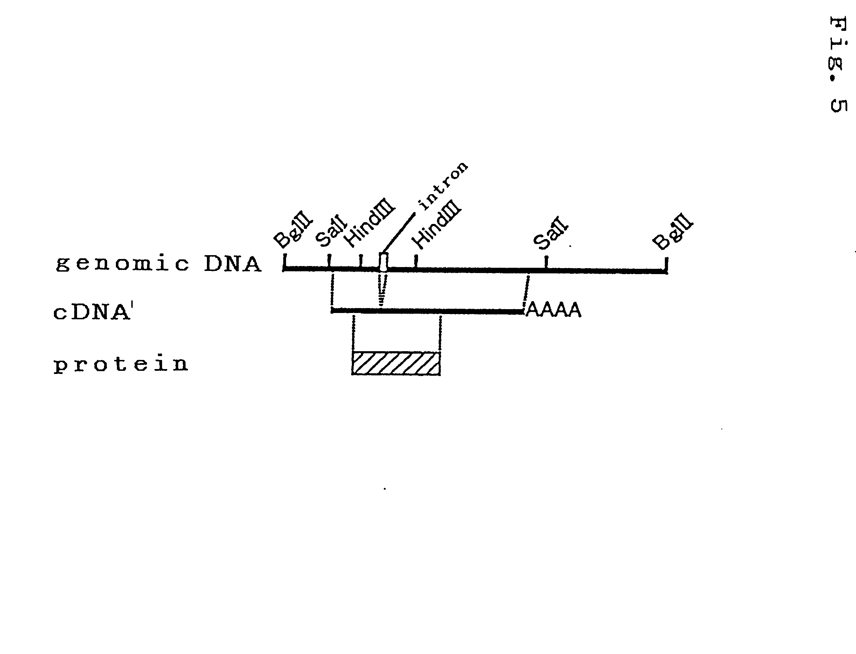

To examine a region appropriately usable as the above-mentioned probe, the present inventors have compared the amino acid sequence of the protein encoded by the gene anaurl^{S} of the present invention (SEQ ID NO. 4 in the Sequence Listing) and the amino acid sequence of the protein encoded by the gene afaurl^{s} of the present invention (SEQ ID NO. 5 in the Sequence Listing) with the amino acid sequence of the protein encoded by an aureobasidin sensitive gene (scaurl^{S}) originating in *S. cerevisiae* (SEQ ID NO. 6), the amino acid sequence of the protein encoded by another aureobasidin sensitive gene (spaurl²) originating in *Schizo. pombe* (SEQ ID NO. 7) and the amino acid sequence of the protein encoded by a gene regulating aureobasidin sensitivity (caaurl) originating in *C. albicans* (SEQ ID NO. 8), each described in Canadian Patent No. 2124034. As a result, no homology is observed as the whole. However, it has been revealed for the first time that there is a characteristic sequence having been conserved in common in these heterogenous genes regulating aureobasidin sensitivity. This conversed sequence has been very well conserved (homology: 80% or above) and is composed of at least eight amino acid residues, which corresponds to a sufficiently long length to be used as a probe. Fig. 4 shows a comparison among the amino acid sequences represented by SEQ ID NOs. 4 to 8 wherein three sequences (Box-1 to Box-3) named "Box sequences" by the inventors correspond to the conserved sequence. Thus, a gene regulating aureobasidin sensitivity and originating in a mold or its functional derivative can be cloned by using a primer or a probe constructed from the amino acid sequence of Box 1, 2 or 3 respectively represented by SEQ ID NOs. 9, 10 or 11 in the Sequence Listing.

The amino acid sequences given in five rows in Fig. 4 correspond respectively to SEQ ID NO. 4 (the top row), SEQ ID NO. 5 (the second row), SEQ ID NO. 6 (the third row), SEQ ID NO. 7 (the fourth row) and SEQ ID NO. 8 (the bottom row).

The target gene encoding the protein regulating aureobasidin sensitivity or its functional derivative may be obtained by hybridization in, for example, the following manner. First, chromosomal DNAs obtained from the target gene source or cDNAs constructed from mRNAs with the use of a reverse transcriptase are connected to a plasmid or a phage vector in accordance with the conventional method and introduced into a host to thereby prepare a library. After incubating this library on a plate, the colonies or plaques thus formed are transferred onto a nitrocellulose or nylon membrane and the DNAs are denatured and thus immobilized on the membrane. This membrane is incubated in a solution containing a probe which has been preliminarily labeled with radio isotope ³²p, etc. (The probe to be used herein may be a gene encoding the amino acid sequence represented by SEQ ID NO. 4 in the Sequence Listing or a part of the same. For example, use can be made of the gene represented by SEQ ID NO. 3 in the Sequence Listing or a part of the same. It is appropriate to use therefor a base sequence which is composed of at least 15 bases and encodes one of the amino acid sequences represented by SEQ ID NOs. 9 to 11 in the Sequence Listing or a part of the same.) Thus DNA hybrids are formed between the DNAs on the membrane and the probe. For example, the membrane having the DNAs immobilized thereon is hybridized with the probe in a solution containing 6 x SSC, 1% of sodium lauryl sulfate, 100 µg/ml of salmon sperm DNA and 5 x Denhardt's solution (containing bovine serum albumin, polyvinylpyrolidone and Ficoll each at a concentration of 0.1%) at 65°C for 20 hours. After the completion of the hybridization, nonspecifically adsorbed matters are washed away and clones forming hybrids with the probe are identified by autoradiography, etc. Into the clone thus obtained, a gene encoding the target protein has been included.

It is confirmed whether or not the obtained gene is the one encoding the target protein regulating aureobasidin sensitivity or its functional derivative, after the DNA sequence of the obtained gene is identified by, for example, the following method.

A clone obtained by the hybridization may be sequenced in the following manner. When the recombinant all is *Escherichia coli,* it is incubated in a test tube, etc. and the plasmid is extracted by a conventional method. Then it is cleaved with restriction enzymes and an insert thus excised therefrom is subcloned into an M13 phage vector, etc. Next, the base sequence is identified by the dideoxy method. When the recombinant is a phage, the base sequence can be identified fundamentally by the same steps. These fundamental experimental procedures to be used from the cell culture to the DNA sequencing are described in, for example, Molecular Cloning, A Laboratory Manual, T. Maniatis et al., Cold Spring Harbor Laboratory Press (1982).

To confirm whether or not the obtained gene is the one encoding the target protein regulating aureobasidin sensitivity or its functional derivative, the amino acid sequence thus identified is compared with the amino acid sequence represented by SEQ ID NO. 4 in the Sequence Listing to thereby know the protein structure and amino acid sequence homology.

To examine whether or not the obtained gene sustains a sensitivity or resistance to aureobasidin, the obtained gene is transformed into sensitive cells and the aureobasidin sensitivity of the transformed cells thus obtained is determined to thereby reveal the activity of the gene. Alternatively, the activity can be determined by transforming the obtained gene into cells from which the activity has been eliminated by disrupting or mutating the gene regulating aureobasidin sensitivity. It is preferable that the above-mentioned gene to be transformed contains sequences required for the expression (promoter, terminator, etc.) in the upstream and/or downstream of the gene so as to enable the expression in the cells transformed.

When the obtained gene fails to contain the whole region encoding the protein regulating aureobasidin sensitivity or its functional derivative, the base sequence of the whole region encoding the protein regulating aureobasidin sensitivity or its functional derivative which is hybridizable with the gene of the present invention encoding the protein regulating aureobasidin sensitivity or its functional derivative can be obtained by preparing synthetic DNA primers on the basis of the gene thus obtained, amplifying the missing region by PCR or further screening a DNA library or a cDNA library with the use of a fragment of the obtained gene as a probe.

For example, a cDNA molecule having the restriction enzyme map of Fig. 3, which contains a gene (afaurl^{S}) of *A. fumigatus* being comparable in function to the gene anaurl^{s}, can be obtained by screening a cDNA library of a pathogenic fungus A. *fumigatus* with the use of a DNA fragment of the PstI-EcoRI fragment (921 bp) of Fig. 2 as a probe. This gene has a base sequence represented by SEQ ID NO. 12 in the Sequence Listing and the amino acid sequence of a protein encoded by this gene, which is estimated on the basis of this base sequence, is the one represented by SEQ ID NO. 5 in the Sequence Listing. When the genes anaurl^{S} and afaurl^{S} are compared, a homology of 87% is observed at the amino acid level. Further, genomic DNAs prepared from *Aspergillus niger* (hereinafter referred to simply as *A. niger*) and Aspergillus oryzae (hereinafter referred to simply as *A. oryzae*) are subjected to the Southern blotting analysis with the use of a DNA fragment of the gene anaurl^{s} as a probe. As a result, it is revealed that genes regulating aureobasidin sensitivity occur in *A. niger* and *A. oryzae.* It is also possible to isolate genes regulating aureobasidin sensitivity from molds other than those belonging to the genus *Aspergillus*, for example, ones of the genus *Penicillium*.

The third aspect of the invention relates to the above-mentioned nucleic acid probe, i.e., an oligonucleotide which is composed of at least 15 bases and hybridizable with a gene regulating aureobasidin sensitivity, for example, a DNA fragment having a restriction enzyme map of Fig. 1, 2 or 3.

This nucleic acid probe is applicable to in situ hybridization, the confirmation of a tissue wherein the above-mentioned gene is expressed, the confirmation of the existence of a gene or mRNA in various vital tissues, etc. This nucleic acid probe can be prepared by ligating the above-mentioned gene or its fragment to an appropriate vector, introducing it into a bacterium followed by replication, extracting with phenol, etc. from a disrupted cell solution, cleaving with restriction enzymes capable of recognizing the ligation site with the vector, electrophoresing and excising from the electrophoresis gels. Alternatively, this nucleic acid probe can be prepared by a chemical synthesis with the use of a DNA synthesizer or gene amplification techniques by PCR on the basis of each of the base sequences represented by SEQ ID NOs. 1, 3 and 12 in the Sequence Listing. Examples of sequences appropriately usable as this nucleic acid probe include base sequences which are composed of at least 15 bases and encode the amino acid sequences represented by SEQ ID NOs. 9 to 11 in the Sequence Listing or a part of the same. To elevate the detection sensitivity, the nucleic acid probe may be labeled with a radioisotope or a fluorescent substance.

The fourth aspect of the invention relates to the antisense DNA of the above-mentioned gene represented by SEQ ID NO.13, while the fifth aspect relates to the antisense RNA thereof represented by SEQ ID NO. 14. By introducing this antisense DNA or antisense RNA into cells, the expression of the gene regulating aureobasidin sensitivity can be controlled.

As the antisense DNA to be introduced, use can be made of, for example, the corresponding antisense DNAs of the genes regulating aureobasidin sensitivity represented by SEQ ID NOs. 1, 3 and 12 in the Sequence Listing or a part of the same. SEQ ID NO. 13 in the Sequence Listing shows an example of such an antisense DNA which corresponds to the sequence of the antisense DNA of the gene regulating aureobasidin sensitivity represented by SEQ ID NO. 1 in the Sequence Listing. As the antisense DNA, it is also possible to use fragments obtained by appropriately cleaving these antisense DNAs or DNAs synthesized on the basis of the sequences of these antisense DNAs.

As the antisense RNA to be introduced, use can be made of, for example, the corresponding antisense RNAs of the genes regulating aureobasidin sensitivity represented by SEQ ID NOs. 1, 3 and 12 in the Sequence Listing or a part of the same. SEQ ID NO. 14 in the Sequence Listing shows an example of such an antisense RNA which corresponds to the sequence of the antisense RNA of the gene regulating aureobasidin sensitivity represented by SEQ ID NO. 1 in the Sequence Listing. As the antisense RNA, it is also possible to use fragments obtained by appropriately cleaving these antisense RNAs or RNAs synthesized on the basis of the sequences of these antisense RNAs. For example, use can be made of an RNA prepared by using the corresponding antisense RNA of the gene regulating aureobasidin sensitivity represented by SEQ ID NO. 1 or 3 in the Sequence Listing and treating it with RNA polymerases in an *in vitro* transcription system.

The antisense DNA and antisense RNA can be chemically modified so as to make them hardly degradable *in vivo* and enable them to pass through cell membrane. A substance capable of inactivating mRNA such as a ribozyme may be bound thereto. The antisense DNA and antisense RNA thus prepared are usable in the treatment of various diseases such as mycosis in association with an increase in the content of the mRNA which encodes the gene regulating aureobasidin sensitivity or its functional derivative.

The sixth aspect of the invention relates to a recombinant plasmid wherein the gene of the first aspect which encodes a protein regulating aureobasidin sensitivity or its functional derivative and originates in a mold, has been integrated into an appropriate vector. For example, a plasmid wherein an aureobasidin resistant gene has been integrated into an appropriate yeast vector is highly useful as a selective marker gene, since it makes it easy to select a transformant showing the drug resistance against aureobasidin.

Also, a recombinant plasmid can be stably carried by *Escherichia coli*, etc. Examples of the vector usable therefor include pUC118, pWH5, pAU-PS, Traplex119 and pTB118.

It is also possible to transform a mold by ligating the gene of the first aspect which encodes a protein regulating aureobasidin sensitivity or its functional derivative and originates in a mold to an appropriate vector. When a plasmid such as pDHG25 [Gene, 98, 61 - 67 (1991)) is employed as the vector, the DNA introduced into the mold can be maintained therein in the state of the plasmid. When a plasmid such as pSa23 [Agricultural and Biological Chemistry, 51; 2549 - 2555 (1987)] is employed as a vector, the DNA can be stably maintained in the state of having been integrated into the chromosome of the mold. It is furthermore possible to give a recombinant plasmid for gene expression by reducing the gene of the present invention into the open reading frame (ORF) alone by cleaving it with appropriate restriction enzymes and by ligating it to an appropriate vector. To construct the plasmid for expression, use can be made of a plasmid such as pTV118, etc. (when *Escherichia coli* is employed as the host), pYE2, etc. (when a yeast is employed as the host), pMAMneo, etc. (when mammal cells are employed as a host) or pTAex3, etc. (when a mold is employed as the host) as the vector.

The seventh aspect of the invention relates to a transformant obtained by introducing the above-mentioned recombinant plasmid into an appropriate host. As the host, use can be made of *Escherichia coli*, yeasts, molds and mammal cells. *Escherichia coli* JM109 transformed by a plasmid pANAR1 which had the gene anaurl^{s} integrated thereinto was named Escherichia coli JM109/pANAR1 and has been deposited at National Institute of Bioscience and Human-Technology, Agency of Industrial Science and Technology under the accession number FERM BP-5180.

The eighth aspect of the invention relates to a process for producing a protein regulating aureobasidin sensitivity or its functional derivative. This process comprises incubating a transformant having the recombinant expression plasmid of the sixth aspect which contains a gene encoding this protein or its functional derivative, in an appropriate nutritional medium, recovering and purifying the protein thus expressed from the cells or the medium. To express the gene encoding this protein, use is made of *Escherichia coli*, a yeast, a mold or mammal cells as the host.

The ninth aspect of the invention relates to a protein regulating aureobasidin sensitivity or its functional derivative. Examples thereof include those encoded by the above-mentioned genes anaurl^{R}, anaurl^{S} and afaurl^{S} and having amino acid sequences represented respectively by SEQ ID NOs. 2, 4 and 5.

As a matter of course, these proteins may have at least one modification selected from among replacement, insertion and deletion by chemical, physical or genetic engineering techniques. It is also possible to construct an antibody against a protein regulating aureobasidin sensitivity by using the proteins having the amino acid sequences represented by SEQ ID NOs. 2, 4 and 5 or a peptide fragment of a region corresponding to a part of such an amino acid sequence as an antigen.

The tenth aspect of the invention relates to a protein capable of imparting aureobasidin resistance wherein at least the amino acid Gly at the position 275 in the gene imparting aureobasidin sensitivity represented by SEQ ID NO. 4 in the Sequence Listing has been replaced by another amino acid. This invention also involves functional derivatives of the same obtained by introducing at least one modification selected from among replacement, insertion and deletion by chemical, physical or genetic engineering techniques thereinto without any injury to the biological activity thereof. The protein of the present invention capable of imparting aureobasidin resistance may be appropriately prepared genetic engineeringly by using DNAs encoding the proteins capable of imparting aureobasidin resistance represented by SEQ ID NOs. 3 and 12 in the Sequence Listing. Its biological activity can be determined by measuring the activity thereof of converting aureobasidin sensitive calls into aureobasidin resistant cells.

The eleventh aspect of the invention relates to a DNA encoding the protein of the tenth aspect capable of imparting aureobasidin resistance. It also involves DNAs obtained by introducing at least one modification selected from among replacement, insertion and deletion of nucleotide(s) into the above-mentioned DNA. Such a modification may be easily effected by a site-specific mutagenesis. These modified DNAs are employed in order to produce mutated proteins.

The nucleic acid probe can also be used in a method for detecting a gene regulating aureobasidin sensitivity by hybridization. Examples of the nucleic acid probe usable herein include oligonucleotides which are composed of at least 15 bases and hybridizable selectively with the DNAs represented by SEQ ID NOs. 1, 3 and 12 in the Sequence Listing and fragments thereof. It is appropriate to use therefor base sequences which encode the amino acid sequences represented by SEQ ID NOs. 9 to 11 in the Sequence Listing or a part of the same and consist of at least 15 bases. By using such a nucleic acid probe, DNAs or RNAs extracted from the target organism are subjected to Southern hybridization or Northern hybridization to thereby give the gene of the target organism regulating aureobasidin sensitivity. The nucleic acid probe is also usable in the confirmation of a tissue wherein the above-mentioned gene can be expressed, or the confirmation of the existence of the gene or mRNA in various vital tissues by in situ hybridization.

This nucleic acid probe can be prepared by ligating the above-mentioned gene or its fragment to an appropriate vector, introducing it into a bacterium followed by replication, extracting with phenol, etc. from a disrupted cell solution, cleaving with restriction enzymes capable of recognizing the ligation site with the vector, electrophoresing and excising from the gel. Alternatively, this nucleic acid probe can be prepared by a chemical synthesis with the use of a DNA synthesizer or gene amplification techniques by PCR on the basis of each of the base sequences represented by SEQ ID NOs. 1, 3 and 12 in the Sequence Listing. To elevate the detection sensitivity in use, the nucleic acid probe may be labeled with a radioisotope or a fluorescent substance.

### [Brief Description of the Drawings]

[Fig. 1]
   Diagram showing the restriction enzyme map of the genomic DNA of a gene anaurl^{R} regulating aureobasidin sensitivity.
[Fig. 2]
   Diagram showing the restriction enzyme map of the cDNA of a gene anaurl^{s} regulating aureobasidin sensitivity.
[Fig. 3]
   Diagram showing the restriction enzyme map of the cDNA of a gene afaurl^{s} regulating aureobasidin sensitivity.
[Fig. 4]
   Diagram showing a comparison among the amino acid sequences of proteins encoded by genes regulating aureobasidin sensitivity.
[Fig. 5]
   Diagram showing a relation among the genomic DNA, cDNA and protein of a gene anaurl regulating aureobasidin sensitivity.
[Fig. 6]
   Diagram showing the results of Northern hybridization of genes regulating aureobasidin sensitivity of *A. nidulans* and *A. fumigatus*.
[Fig. 7]
   Diagram showing the results of Southern hybridization which indicate the detection of genes regulating aureobasidin sensitivity of *A. niger* and *A. oryzae*.

### [Examples]

To further illustrate the present invention in greater detail, and not by way of limitation, the following Examples will be given.

### Example 1: Cloning of gene anaur1 regulating aureobasidin sensitivity and originating in A. nidulans

### 1-a) Isolation of aureobasidin resistant mutant of A. nidulans

A strain *A. nidulans* FGSC89 showing a sensitivity to aureobasidin at 5 µg/ml was inoculated into an SD slant (containing 1% of polypeptone S, 2% of glucose and 2% of agar) and incubated therein at 30°C for 7 days. After suspending in 5 ml of a 0.1% Tween 80 solution containing 0.8% of NaCl, the suspension was filtered through a glass filter (3G3 type) and the obtained filtrate was used as a conidium suspension. This conidium suspension was UV-radiated for 5 minutes, thus effecting mutagenesis. Under these conditions, the rate of survival was about 25%. After blocking off the light for 30 minutes or longer, the conidia were inoculated into an SD plate and incubated at 30°C for 4 days. The conidia thus formed were collected with a glass filter, further inoculated into Cz+bi medium [containing 4.9% of Czapek solution agar (manufactured by Difco), 200 µg/ml of arginine and 0.02 µg/ml of biotin] containing 5 µg/ml of aureobasidin and incubated therein at 30°C. After 2 or 3 days, eight aureobasidin resistant colonies were obtained. Although these cells showed a resistance to 80 µg/ml of aureobasidin, they were the same as the parent strain in the sensitivities to amphotericin B, cycloheximide and clotrimazole. Thus it was estimated that the resistance thus acquired was not a multiple drug resistance but one specific to aureobasidin.

### 1-b) Preparation of genomic library of aureobasidin resistant strain

From a strain R1 showing a particularly high resistance from among the aureobasidin resistant strains, genomic DNAs were extracted and purified in the following manner. After incubating in a PD medium [containing 2.4% of potato dextrose broth (manufactured by Difco)] under shaking at 30°C for 2 days, the hyphae were collected with a glass filter (3G1 type) and washed with distilled water. Then the cells were dehydrated and suspended in 20 ml of a protoplast generation solution [containing 20 mg/ml of Yatalase (manufactured by Ozeki Shuzo), 0.8 M of NaCl and 10 mM of a sodium phosphate buffer, pH 6.0]. Then the suspension was slowly stirred at 30°C overnight to thereby generate protoplasts. The suspension was filtered through a glass filter (3G2 type) and thus the protoplasts were collected into the filtrate and then harvested by centrifuging at 2,000 rpm for 5 minutes. After washing with 0.8 M NaCl twice, the protoplasts were suspended in 2 ml of a TE solution (containing 10 mM of Tris-HCl and 1 mM of EDTA, pH 8.0) and 2 ml of a lysis solution (containing 2% of SDS, 0.1 M of NaCl, 10 mM of EDTA and 50 mM of Tris-HCl, pH 7.0) was added thereto. After slowly stirring, the mixture was maintained at room temperature for 15 minutes and then centrifuged at 3,500 rpm for 10 minutes followed by the recovery of the supernatant. Then an equivalent amount of a mixture of phenol/chloroform/isoamyl alcohol (25/24/1) was added thereto and the mixture in the tube was gently mixed and centrifuged at 3,000 rpm for 5 minutes followed by the recovery of the upper liquid layer. Next, 2.5 times by volume as much ethanol at -20°C was added thereto. The resulting mixture was allowed to stand at -80°C for 10 minutes and then centrifuged at 3,500 rpm for 15 minutes. The DNAs thus precipitated were dried. Then 0.5 ml of a TE solution and 2.5 µl of an RNase A solution (20 mg/ml) were added thereto and the mixture was maintained at 37°C for 30 minutes.

After adding 0.5 ml of phenol/chloroform/isoamyl alcohol, the obtained mixture was gently mixed and centrifuged at 10,000 rpm for 5 minutes followed by the recovery of the upper layer. This procedure was repeated once. After adding 0.5 ml of chloroform/isoamyl alcohol (24/1), the obtained mixture was gently mixed and centrifuged at 10,000 rpm for 5 minutes followed by the recovery of the supernatant. Then 0.05 ml of 5 M NaCl and 0.5 ml of isopropyl alcohol were added and the mixture was allowed to stand at -80°C for 10 minutes and centrifuged at 10,000 rpm for 15 minutes to thereby collect DNAs.

Eight µg of the genomic DNAs thus purified were partially digested by treating with 4 U of a restriction enzyme BamHI at 37°C for 15 minutes. After deprotenization with phenol/chloroform, the DNA was recovered by ethanol precipitation. The DNAs were electrophoresed on a 0.8% agarose gel and DNAs in a region of from 3 to 15 kb were extracted and purified. The DNAs thus obtained were ligated to a vector pDHG25 [Gene, 98, 61 - 67 (1991)], which had been completely digested with BamHI, with the use of a DNA ligation kit (manufactured by Takara Shuzo Co., Ltd.). Then *Escherichia coli*, HB101 was transformed thereby so as to prepare a genomic library of the resistant strain. The *E. coli* cells containing this genomic library were cultured in 50 ml of an LB medium (containing 1% of bactotrypton, 0.5% of bacto yeast extract and 0.5% of sodium chloride) containing 100 µg/ml of ampicillin at 37°C overnight. Next, plasmids were recovered and purified from the *E. coli* cells.

### 1-c) Expression and cloning of aureobasidin resistant gene anaurl^{R}

The plasmid originating in the genomic library of the aureobasidin resistant strain thus obtained was transformed into a strain *A. nidulans* FGSC89 by the following method. Namely, *A. nidulans* was incubated in a PD medium under shaking at 30°C for 2 days. Then the hyphae were collected by filtering the culture broth through a glass filter (3G1 type) and washed with sterilized water. After sufficiently dehydrating, the cells were suspended in 10 ml of a protoplast generation solution. After reacting by slowly shaking at 30°C for about 3 hours, the cell suspension was filtered through a glass filter 3G3. Then the filtrate was centrifuged at 2,000 rpm for 5 minutes to thereby collect the protoplasts therein. The collected protoplasts were washed with 0.8 M NaCl twice and suspended in Sol 1 (containing 0.8 M of NaCl, 10 mM of CaCl₂ and 10 mM of Tris-HCl, pH 8.0) in such a manner as to give a protoplast concentration of 2 × 10⁸/ml. Then 0.2 time by volume as much Sol 2 [containing 40% (w/v) of PEG4000, 50 mM of CaCl₂ and 50 mM of Tris-HCl, pH 8.0] was added thereto and well mixed.

10 µg of the plasmid originating in the genomic library was added to a 0.2 ml portion of the protoplast suspension. After mixing well, the mixture was allowed to stand in ice for 30 minutes and then 1 ml of Sol 2 was added thereto. After mixing well, the mixture was allowed to stand at room temperature for 15 minutes and then 8.5 ml of Sol 1 was added thereto. After mixing well, the mixture was centrifuged at 2,000 rpm for 5 minutes to thereby collect the protoplasts. 0.2 ml of Sol 1 was added thereto and the resulting mixture was placed on the center of a minimum medium plate (containing 4.9% of Czapek solution agar, 0.8 M of NaCl and 0.02 µg/ml of biotin) containing 5 µg/ml of aureobasidin. Next, 5 ml of a soft agar medium (containing 3.5% of Czapek-Dox broth, 0.8 M of NaCl, 0.02 µg/ml of biotin and 0.5% of agar) was layered thereon followed by incubation at 30°C for 3 to 5 days. It was considered that the colonies growing on this plate carried a plasmid containing an aureobasidin resistant gene.

Thus about 70 colonies were formed on the aureobasidin-containing medium. These colonies were transplanted into 20 ml of a Cz+Bi medium and incubated at 30°C for 2 days. Then DNA was recovered and purified from the cells thus propagated in accordance with the method for the extraction and purification of DNA described in Example 1-b). A strain *E. coli* HB101 was transformed by this DNA and spread on an LB plate containing 100 µg/ml of ampicillin. Then a plasmid DNA was prepared from the *E. coli* colonies thus formed. This plasmid contained a DNA of 12 kb and was named pR1-1. Fig. 5 shows the restriction enzyme map of the 12 kb DNA contained in pR1-1. To further specify the resistant gene region, the DNA fragment of 12 kb was digested into fragments of various sizes with restriction enzymes. Next, these fragments were cloned into a vector pDHG25. Plasmids containing various DNAs were transformed into a strain *A. nidulans* FGSC89 so as to confirm whether the aureobasidin resistance could be thus acquired or not. As a result, it was revealed that the activity of imparting aureobasidin resistance resided in a fragment Bgl II (5.8 kb). Thus it was clarified that the gene anaurl^{R} was located in this fragment. Fig. 1 shows the restriction enzyme map of this DNA fragment containing the aureobasidin resistant gene anaurl^{R}. This fragment was subcloned into a vector pUC118 and the obtained plasmid was named pUR1. By using this plasmid, the DNA sequence of the DNA was identified. SEQ ID NO. 1 in the Sequence Listing shows this base sequence. As this DNA sequence indicates, the gene anaur1^{R} is one composed of two exon regions containing an intron. It has been revealed that this gene encodes a protein having the amino acid sequence represented by SEQ ID NO. 2 in the Sequence Listing.

### 1-d) Cloning of aureobasidin sensitive gene anaurl^{S}

To obtain the cDNA of the aureobasidin sensitive gene from normal cells of A. *nidulans*, total RNAs were first extracted from a strain *A. nidulans* FGSC89. Namely, this strain was incubated in 200 ml of a PD medium and the cells were collected with the use of a glass filter (3G1 type). After sufficiently dehydrating, the cells were quickly frozen with liquid nitrogen. Then the frozen cells were powdered in a mortar and total RNAs (2.6 mg) were extracted and purified with the use of an RNA extraction kit (manufactured by Pharmacia). From 1 mg of these RNAs, 12.8 µg of poly(A)⁺RNAs were prepared by using Oligotex-dT30 <Super> (manufactured by Takara Shuzo Co., Ltd.). By using 5 µg of the poly(A)⁺RNAs, cDNAs were synthesized with the use of a Takara cDNA synthesizing kit (manufactured by Takara Shuzo Co., Ltd.). The cDNAs thus synthesized were ligated to a λ phage vector λSH1ox™ (manufactured by Novagen, Inc.) and subjected to in vitro packaging with the use of Phage Maker™ System, Phage Pack Extract (manufactured by Novagen, Inc.) to thereby construct a cDNA library. This cDNA library was infected in a host strain *E. coli* ER1647. After mixing with top agarose (an LB medium containing 0.7% of agarose), it was layered on an LB plate and incubated at 37°C overnight to thereby form plaques. The plaques thus formed were transferred onto a nylon membrane (Hybond-N, manufactured by Amersham) and subjected to plaque hybridization. As a probe, use was made of a DNA fragment of 2.6 kb obtained by cleaving the plasmid pUR1 obtained in Example 1-c) with PstI and SalI. This DNA fragment was labeled with [α-³²P]dCTP by using a random primer DNA labeling kit (manufactured by Takara Shuzo Co., Ltd.) and employed as a probe in the hybridization. As the result of screening of 4 × 10⁵ plaques, 8 phage clones hybridizable with the probe were obtained. Next, these phages were subjected to automatic subcloning in *E. coli* to thereby give *E. coli* strains having plasmids wherein a cDNA-containing region had been automatically subcloned. The plasmids were purified from these strains and the cDNAs were compared in length. Thus pS15 having the longest cDNA (2.9 kb) was selected and subcloned into pUC118 followed by the identification of the DNA sequence. This plasmid was named pANAR1. An *E. coli* strain transformed by pANAR1 was named Escherichia coli JM109/pANAR1 and has been deposited at National Institute of Bioscience and Human-Technology, Agency of Industrial Science and Technology under the accession number FERM BP-5180. Fig. 2 shows the restriction enzyme map of this cDNA. The DNA base sequence thereof is represented by SEQ ID NO. 3 in the Sequence Listing. As this base sequence indicates, the gene anaurl^{S} encodes a protein having the amino acid sequence represented by SEQ ID NO. 4 in the Sequence Listing. A comparison with the resistant gene anaurl^{R} has revealed that the base G at the position 1218 in SEQ ID NO. 3 has been mutated into T and, at the amino acid level, the amino acid glycine at the position 275 has been converted into valine. It has been further clarified that the genomic DNA has one intron (56 bp). Fig. 5 shows the relation between the genomic DNA and cDNA.

### Example 2: Confirmation and cloning of gene afaurl^{S} carried by A. fumigatus

### 2-a) Detection of gene afaurl^{S} by Northern hybridization

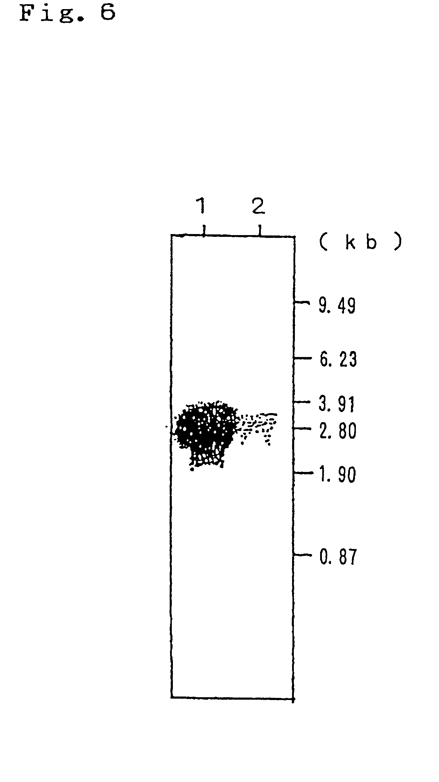

From a strain *A. fumigatus* TIMM1776, poly(A)⁺RNAs were extracted and purified by the same method as the one of Example 1-d). The poly(A)⁺RNAs (1 µg) of A. *fumigatus* and *A. nidulans* were separated by electrophoresing on a 1.2% agarose gel containing formaldehyde and transferred onto a nylon membrane. After fixing, hybridization was effected with the use of a HindIII fragment (741 bp) of the cDNA of the gene anaurl^{s} labeled with [α-³²P]dCTP as a probe. After hybridizing at 60°C overnight, the mixture was washed at 60°C with 0.5 x SSC and 0.1% SDS. In Fig. 5, the lanes 1 and 2 show the results of the hybridization of the poly(A)⁺RNAs obtained from *A. nidulans* and A. *fumigatus* respectively. As Fig. 6 clearly shows, autoradiography of the hybridization revealed that *A. fumigatus* and *A. nidulans* both had the aureobasidin sensitive genes of the same size. However, the band of A. *fumigatus* was very weak, which indicates that the homology between these genes is not so high.

### 2-b) Cloning of gene afaurl^{S} carried by A. fumigatus

By using the poly(A)⁺RNAs of *A. fumigatus* purified in Example 2-a), a cDNA library of *A. fumigatus* was prepared in accordance with the method for the preparation of a cDNA library described in Example 1-d). The above-mentioned library was screened under the same hybridization conditions as those of Example 2-a) with the use of a PstI-EcoRI fragment (921 bp) of the cDNA of *A. nidulans* as a probe. Thus eight phage clones were obtained. From these phase clone, the cDNA was recovered in the form of a plasmid by the method described in Example 1-d). The plasmids were purified and the cDNAs were compared in length. Thus the plasmid having the longest cDNA (2.9 kb) among them was selected. This cDNA was subcloned into pUC118 and the DNA sequence was identified. This base sequence, which is represented by SEQ ID NO. 12 in the Sequence Listing, indicates that this gene encodes a protein having the amino acid sequence represented by SEQ ID NO. 5 in the Sequence Listing. A comparison between the amino acid sequence of the afaurl^{s} protein of the strain *A. fumigatus* TIMM1776 with that of the anaurl^{s} protein of the strain *A. nidulans* FGSC 89 indicated that they had a high homology (87%).

### Example 3: Confirmation of genes regulating aureobasidin sensitivity carried by A. niger and A. oryzae

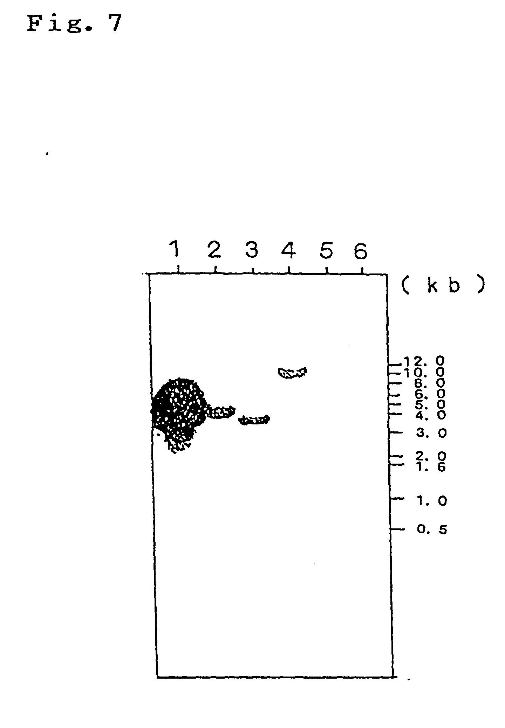

Genomic DNAs were extracted and purified from strains *A. fumigatus* TIMM1776, *A. niger* FGSC805 and *A. oryzae* IFO5710 in accordance with the method described in Example 1-b). Further, genomic DNAs were extracted and purified from yeast strains *S. cerevisiae* DKD-5D and *Schizo. pombe* JY745 in accordance with the method of P. Philippsen et al. [Methods in Enzymology, 194, 169 - 175 (1991)]. 5 µg portions of the genomic DNAs of *A. nidulans, A. fumigatus, A. niger, A. oryzae, S*. *cerevisiae* and *Schizo. pombe* were cleaved with a restriction enzyme PstI, separated by electrophoresing on a 0.8% agarose gel, transferred onto a nylon membrane and fixed. Next, Southern hybridization was effected by using a PstI-EcoRI fragment of the cDNA of the anaurl^{S} gene labeled with [α-³²P]dCTP as a probe. The hybridization was effected under the same conditions as those described in Example 2-a). Fig. 7 shows the autoradiogram of the hybridization. As Fig. 7 clearly shows, genes regulating aureobasidin sensitivity occur in *A. niger* and *A. oryzae* too. It has been also revealed that the DNA of *A. nidulans* is not hybridizable with the aureobasidin sensitive genes of the yeasts *S*. *cerevisiae* and *Schizo. pombe*. In Fig. 7, the lanes 1, 2, 3, 4, 5 and 6 show the results of the Southern hybridization of the genomic DNAs of *A. nidulans, A. fumigatus, A. niger, A. oryzae, S. cerevisiae* and *Schizo. pombe* respectively.

### [Effects of the Invention]

The present invention provides a novel protein regulating aureobasidin sensitivity and originating in a mold of the genus Aspergillus and a gene encoding this protein, i.e., a gene regulating aureobasidin sensitivity. These substances are useful in the diagnosis and treatment of diseases, such as mycosis, caused by organisms carrying the gene. The present invention further provides the antisense DNA and antisense RNA of the above-mentioned gene, and a process for producing a protein regulating aureobasidin sensitivity with the use of a transformant having this gene introduced thereinto. These substances are also useful in the diagnosis and treatment of diseases exemplified by mycosis.

### Sequence Listing

SEQ ID NO : 1
   SEQUENCE LENGTH : 4140
   SEQUENCE TYPE : nucleic acid
   STRANDEDNESS : double
   TOPOLOGY : linear
   MOLECULE TYPE : Genomic DNA
   SEQUENCE DESCRIPION : 
AGATCTGTGG CTTCCGGTTG GCTACTTGTA ACCAACTGAT GGTCAGATGG ATCTGCCGTC 60
TGTTTTGATT TGAATTTTCC CTGCTCATTC TGATTCTGTG AGAGGCTGCA TTCATTATCA 120
CATCTCATAC CCGGCGCCTG CGACTTCGGT CACCTCTGCG GTCTGGCGGT TACCGGGGTC 180
CGTCTGAGAC TCGTCAGTCA GCCATTCGAG TATGCGAACT CTGACTTTGC TCACCTAAGA 240
GTTTGCACGA GATGCCGAAA TCCTCCTCGA GTAGAGTTTG CAAGGCTTGA ACCTTGGTCC 300
TTGAAGCCCG AAAGTGGCTC AGTAGTGGGA TCGATAGTCT GGTTGTTGAA GATTTTCTCC 360
TCCACCTTAC CTATGGCCGC TGGCCTTCTC CACCTTTCAG GCTTTCAGGC ACCCTCGGCT 420
CGGATTCTGT ATCGTCCGGT ACCGAAGCTA GTCCTAGCTA GTCAAAGCTA GTCCAAGCTA 480
GTCTCGTCAA GGTTTGGCGC AGCGCGGTTC CGTGTAAAGT ACAAATTTGA AATACGAATA 540
CGCAGTACTC GCAGCCGGCA CTTCCGCTCA GCCCAGGCTC AGAGGCTAAG GGTGTTGGCG 600
CTTCCTCATC ATCTTCTTCT CGTCGACCTT TTCCTCTTTC TCTCCCTATC GGTGCTTCTC 660
TCCAACCTCA TTCTCAGTCG TTCGCCCATC AGGTTTATAC TCCGGCTCCG TGGCCATCTG 720
CCTCCCTCAC GACCTCCTCG TTCCAGGTTT TCCTCTCGAC TGCTGCGCCC TTGCACTTCG 780
CCTTGCATCA GTGAAACCCC CTGCAACGTG ACGGCTCAAA GACATCCTCG TTTGGCCGCT 840
GGAGACCGGA GCGTGCGCTT CGTTTCGTCT TCTTCGAACC GATCTCAATT TCCCCGCTCG 900
GGTTGACGCC GTCAGCACCC TGCTCGTTGC CTAACGGCTT GTTATTCAAG ACCCCTTTTC 960
TGCCGCTTCC GCGACCGATT TATTCGTCGC CTTCCAACTC TTGTACAATC GGGGGGAAAG 1020
AAAGCAGACG GAGTTCGATC TGGAGGAATT ATAGCTGAGT CTTGCCCGCA AGACTCGCCG 1080
CAACCATGAA TCAAACACTT CCCACGTGGA AGGACCGCAC GCAGAACCAG TTTGGAAAGC 1140
TTCAGATCCA GGTTCCATGG CGGTCCATCC AACTGCTCGT CCCGCATCGC ATGCGGCGGA 1200
AGTTAAGGTC CAAATTGCGC AGTAGAGCGT CTCCTACCTC GTCAATAGCC TCTTTACAGA 1260
 
CGTCGTTATC GCCTGCAGAC ACACTACGAT CGCTCCAAAG CCACCGATGG ACGGTTTACG 1320
ACTTCCAATA TCTGCTTCTG TTGATCGTGG GCATCTTCTC TTTGACCGTT ATCGAGTCGC 1380
CCGGGCCTTT GGGCAAAACG GCCATTTTCT CCATGCTCCT ATTCTCTCTC CTGATCCCTA 1440
TGACCCGCCA GTTCTTCCTC CCGTTTCTGC CGATTGCCGG ATGGCTTCTG TTTTTCTACG 1500
CCTGCCAGTG AGTTAAAAAC AACCCGCTAC CAGACCCCGT GCAGCAGTTA CTCACATATG 1560
CAGGTTCATC CCAAGCGATT GGCGCCCTGC GATTTGGGTT CGTGTCTTGC CTGCACTGGA 1620
GAATATTCTC TACGGCGCAA ACATCAGCAA CATCCTATCC GCTCACCAGA ACGTTGTGCT 1680
TGACGTGCTG GCGTGGCTAC CCTACGGTAT CTGCCACTAT GGCGCTCCGT TTGTGTGCTC 1740
GTTGATCATG TTCATCTTCG GTCCGCCCGG CACTGTTCCC CTTTTCGCGC GCACTTTCGG 1800
CTATATCAGT ATGACTGCGG TTACTATTCA GCTGTTTTTC CCTTGCTCTC CACCTTGGTA 1860
TGAGAATCGC TATGGTCTAG CTCCGGCAGA CTACTCCATC CAAGGTGATC CCGCAGGGCT 1920
TGCCCGCATT GACAAGCTTT TCGGCATCGA CCTTTACACG TCTGTTTTCC ATCAGTCGCC 1980
TGTTGTGTTC GGCGCTTTTC CGTCGCTGCA TGCTGCCGAC TCAACCCTGG CCGCACTTTT 2040
CATGAGTCAT GTTTTCCCCC GCATGAAGCC CGTCTTCGTG ACCTATACTC TATGGATGTG 2100
GTGGGCAACA ATGTACCTCT CACATCACTA TGCGGTCGAT TTGGTTGCGG GTGGTCTCCT 2160
GGCCGCCATT GCTTTCTACT TCGCCAAGAC CCGATTCCTT CCCCGTGTCC AGCTCGACAA 2220
GACCTTCCGT TGGGACTACG ACTATGTGGA ATTCGGCGAG TCTGCCCTGG AGTATGGGTA 2280
TGGTGCAGCT GGCTATGATG GAGACTTCAA TCTCGACAGC GATGAATGGA CTGTTGGTTC 2340
TTCATCCTCC GTCTCCTCAG GCTCCTTGAG TCCCGTTGAC GATCATTACT CATGGGAAAC 2400
CGAGGCACTG ACCTCCCCAC ATACTGATAT TGAGTCCGGC AGGCATACTT TCAGCCCTTG 2460
AGTAGCCACA AACCAAACTC GATACCTGCA TATAGCGATC TCGCTCCTCC TCCACTGCAT 2520
CTATTTACGA GACGGCGTTA GAACATTTCA CGACATTCTG GCTTTATTGC ATCGAGCACA 2580
TTTCGACACA TATATCTTTA ATACCCTTTC TTCGGTGTCC CAGATCATCG GTTCGACCTT 2640
AATGTACCTC GGTCCGAATC CGCCTGGGAT ACTGTTTCTC TTTCCGCCGC ACTTCACTGT 2700
ACATTGCTTG ACATTGCGAA ACCGGGTTGG GCTCGAACGT GGGATGGGTT ATCGCTCATC 2760
GCTACACGCC GTTGCTCCAT CATAATGTTA ATGGACACAA TGGGGCTACG CATCCTGGTG 2820
TTTAGTCCTG GAAGACCATC CGATAACCCC CGTCGGTAAC ACTCGCTTGT CTCGTGTCCA 2880
CCCAGACACT ACTTCAATTC TCACTTCTAT CGTCCGCTAT TACCTTGACC TGGTCGAACC 2940
CATCCTTATT ATTCGTTTCG ACTATGCTAT ATATTTATTT TTACCATTCG TGTCGATCGC 3000
 
TCATACTCTT GGCGCTTGGG ACTGGAAGCA TTTATATTGG AAAAAATCAC GGAATGGGGC 3060
GCCTTTTCTT CTTGCACTTC ACTCGCTGTG CATAGACGGT TTTACATTTC TGCTTTGCAA 3120
TGCATCACGA ACTCTGCATT AGCATATAGA AAGAGGGGAA GGATGGACCT TCTTCTTGAT 3180
TGCTCGCATG GTTTATCCAT TCGCTCAAAG TGGATTACGT CCACATATTA CCCGGGGGCT 3240
ATACACATGG CTACTGTGTT GCTTTCTGAC ATTCGCCGGA CGTGCAAGGT TGGGAGGAGA 3300
GTCTGACGCT GACGGGGCTT GTTGAAGGAT GTTCACGCGT CCCGATTTGA CCCGGCTTCG 3360
ACTAACCTCA GATTCTCGAC TTGTTGGACG GTGACTTGAC TTGCTTGCTA TGGTCTGACG 3420
CTCTCACACC TACCTATCAC ATCCTCCTCA CCTCACAAAT TCCGCTCATG GACACTATCC 3480
TCTTCTTTTC GTTTCCCTTG GATAGTGTGT GTGTGTGTGT GGTTGGGGCA AATTATCCAT 3540
AGCAGCAGTA TTATTAGTTA TAATCCGGTA GTGTTATGAT TTATGAAGGC AACTTGTATA 3600
CTATTGCCAC TTTGTCCATA TCTCTTGCTT GTAATAGAAC TGACATCGCG ACGCTTCGGC 3660
CACGATGCAT ATAAAAACTC TAGTCAACAC GATATTAACA AGCGAAACCA TTACGCTGTA 3720
AACTATTCAG GATCGCCGCG GGCCCATCTG GGACTTGACT GTACTAAATA TGTCCTAAAG 3780
CAAGCAGACT AAATATTTAA CGTGGGATAT TATTCATATA CGCATATGTA TACATAGTCA 3840
TAACAAGCCA AGGGGTGGGT AGGGGTGGGT AATTATTATT TTTTTCGTCC GATACAAGTA 3900
TCCATCCTTA AATGTCCGTG GTCTACTCTT CATAAATCTT AACCCGCTCC GCATACTCCT 3960
TTATCCTCGA GACAAAAGTG TCTTCAATTT CATCGCCACG GCCACCAGCA ACGCGGAGGA 4020
TAAGGTCGTT GAGAGAGGCG CGGCCGAGAA TGTCGACATG GTCGCCTTCA TATTGTTAGC 4080
ATGCGACGTC AGACTGGAAC CAGGAAGGGA AAGGAGAGAG GTACCTGTAT TTGGACCACC 4140
SEQ ID NO : 2
   SEQUENCE LENGTH : 439
   SEQUENCE TYPE : amino acid
   STRANDEDNESS : single
   TOPOLOGY: linear
   MOLECULE TYPE : peptide
   SEQUENCE DESCRIPTION : 
Met Asn Gln Thr Leu Pro Thr Trp Lys Asp Arg Thr Gln Asn Gln
1               5                   10                  15
 
Phe Gly Lys Leu Gln Ile Gln Val Pro Trp Arg Ser Ile Gln Leu
                20                  25                  30
Leu Val Pro His Arg Met Arg Arg Lys Leu Arg Ser Lys Leu Arg
                35                  40                  45
Ser Arg Ala Ser Pro Thr Ser Ser Ile Ala Ser Leu Gln Thr Ser
                50                  55                  60
Leu Ser Pro Ala Asp Thr Leu Arg Ser Leu Gln Ser His Arg Trp
                65                  70                  75
Thr Val Tyr Asp Phe Gln Tyr Leu Leu Leu Leu Ile Val Gly Ile
                80                  85                  90
Phe Ser Leu Thr Val Ile Glu Ser Pro Gly Pro Leu Gly Lys Thr
                95                  100                 105
Ala Ile Phe Ser Met Leu Leu Phe Ser Leu Leu Ile Pro Met Thr
                110                 115                 120
Arg Gln Phe Phe Leu Pro Phe Leu Pro Ile Ala Gly Trp Leu Leu
                125                 130                 135
Phe Phe Tyr Ala Cys Gln Phe Ile Pro Ser Asp Trp Arg Pro Ala
                140                 145                 150
Ile Trp Val Arg Val Leu Pro Ala Leu Glu Asn Ile Leu Tyr Gly
                155                 160                 165
Ala Asn Ile Ser Asn Ile Leu Ser Ala His Gln Asn Val Val Leu
                170                 175                 180
Asp Val Leu Ala Trp Leu Pro Tyr Gly Ile Cys His Tyr Gly Ala
                185                 190                 195
Pro Phe Val Cys Ser Leu Ile Met Phe Ile Phe Gly Pro Pro Gly
                200                 205                 210
Thr Val Pro Leu Phe Ala Arg Thr Phe Gly Tyr Ile Ser Met Thr
                215                 220                 225
Ala Val Thr Ile Gln Leu Phe Phe Pro Cys Ser Pro Pro Trp Tyr
 
                230                 235                 240
Glu Asn Arg Tyr Gly Leu Ala Pro Ala Asp Tyr Ser Ile Gln Gly
                245                 250                 255
Asp Pro Ala Gly Leu Ala Arg Ile Asp Lys Leu Phe Gly Ile Asp
                260                 265                 270
Leu Tyr Thr Ser Val Phe His Gln Ser Pro Val Val Phe Gly Ala
                275                 280                 285
Phe Pro Ser Leu His Ala Ala Asp Ser Thr Leu Ala Ala Leu Phe
                290                 295                 300
Met Ser His Val Phe Pro Arg Met Lys Pro Val Phe Val Thr Tyr
                305                 310                 315
Thr Leu Trp Met Trp Trp Ala Thr Met Tyr Leu Ser His His Tyr
                320                 325                 330
Ala Val Asp Leu Val Ala Gly Gly Leu Leu Ala Ala Ile Ala Phe
                335                 340                 345
Tyr Phe Ala Lys Thr Arg Phe Leu Pro Arg Val Gln Leu Asp Lys
                350                 355                 360
Thr Phe Arg Trp Asp Tyr Asp Tyr Val Glu Phe Gly Glu Ser Ala
                365                 370                 375
Leu Glu Tyr Gly Tyr Gly Ala Ala Gly Tyr Asp Gly Asp Phe Asn
                380                 385                 390
Leu Asp Ser Asp Glu Trp Thr Val Gly Ser Ser Ser Ser Val Ser
                395                 400                 405
Ser Gly Ser Leu Ser Pro Val Asp Asp His Tyr Ser Trp Glu Thr
                410                 415                 420
Glu Ala Leu Thr Ser Pro His Thr Asp Ile Glu Ser Gly Arg His
                425                 430                 435
Thr Phe Ser Pro
SEQ ID NO : 3
   SEQUENCE LENGTH: 2856
   SEQUENCE TYPE: nucleic acid
   STRANDEDNESS : double
   TOPOLOGY: linear
   MOLECULE TYPE: cDNA
   SEQUENCE DESCRIPTION: 
GGTTTATACT CCGGCTCCGT GGCCATCTGC CTCCCTCACG ACCTCCTCGT TCCAGGTTTT 60
CCTCTCGACT GCTGCGCCCT TGCACTTCGC CTTGCATCAG TGAAACCCCC TGCAACGTGA 120
CGGCTCAAAG ACATCCTCGT TTGGCCGCTG GAGACCGGAG CGTGCGCTTC GTTTCGTCTT 180
CTTCGAACCG ATCTCAATTT CCCCGCTCGG GTTGACGCCG TCAGCACCCT GCTCGTTGCC 240
TAACGGCTTG TTATTCAAGA CCCCTTTTCT GCCGCTTCCG CGACCGATTT ATTCGTCGCC 300
TTCCAACTCT TGTACAATCG GGGGGAAAGA AAGCAGACGG AGTTCGATCT GGAGGAATTA 360
TAGCTGAGTC TTGCCCGCAA GACTCGCCGC AACCATGAAT CAAACACTTC CCACGTGGAA 420
GGACCGCACG CAGAACCAGT TTGGAAAGCT TCAGATCCAG GTTCCATGGC GGTCCATCCA 480
ACTGCTCGTC CCGCATCGCA TGCGGCGGAA GTTAAGGTCC AAATTGCGCA GTAGAGCGTC 540
TCCTACCTCG TCAATAGCCT CTTTACAGAC GTCGTTATCG CCTGCAGACA CACTACGATC 600
GCTCCAAAGC CACCGATGGA CGGTTTACGA CTTCCAATAT CTGCTTCTGT TGATCGTGGG 660
CATCTTCTCT TTGACCGTTA TCGAGTCGCC CGGGCCTTTG GGCAAAACGG CCATTTTCTC 720
CATGCTCCTA TTCTCTCTCC TGATCCCTAT GACCCGCCAG TTCTTCCTCC CGTTTCTGCC 780
GATTGCCGGA TGGCTTCTGT TTTTCTACGC CTGCCAGTTC ATCCCAAGCG ATTGGCGCCC 840
TGCGATTTGG GTTCGTGTCT TGCCTGCACT GGAGAATATT CTCTACGGCG CAAACATCAG 900
CAACATCCTA TCCGCTCACC AGAACGTTGT GCTTGACGTG CTGGCGTGGC TACCCTACGG 960
TATCTGCCAC TATGGCGCTC CGTTTGTGTG CTCGTTGATC ATGTTCATCT TCGGTCCGCC 1020
CGGCACTGTT CCCCTTTTCG CGCGCACTTT CGGCTATATC AGTATGACTG CGGTTACTAT 1080
TCAGCTGTTT TTCCCTTGCT CTCCACCTTG GTATGAGAAT CGCTATGGTC TAGCTCCGGC 1140
AGACTACTCC ATCCAAGGTG ATCCCGCAGG GCTTGCCCGC ATTGACAAGC TTTTCGGCAT 1200
CGACCTTTAC ACGTCTGTTT TCCATCAGTC GCCTGTTGTG TTCGGCGCTT TTCCGTCGCT 1260
GCATGCTGCC GACTCAACCC TGGCCGCACT TTTCATGAGT CATGTTTTCC CCCGCATGAA 1320
 
GCCCGTCTTC GTGACCTATA CTCTATGGAT GTGGTGGGCA ACAATGTACC TCTCACATCA 1380
CTATGCGGTC GATTTGGTTG CGGGTGGTCT CCTGGCCGCC ATTGCTTTCT ACTTCGCCAA 1440
GACCCGATTC CTTCCCCGTG TCCAGCTCGA CAAGACCTTC CGTTGGGACT ACGACTATGT 1500
GGAATTCGGC GAGTCTGCCC TGGAGTATGG GTATGGTGCA GCTGGCTATG ATGGAGACTT 1560
CAATCTCGAC AGCGATGAAT GGACTGTTGG TTCTTCATCC TCCGTCTCCT CAGGCTCCTT 1620
GAGTCCCGTT GACGATCATT ACTCATGGGA AACCGAGGCA CTGACCTCCC CACATACTGA 1680
TATTGAGTCC GGCAGGCATA CTTTCAGCCC TTGAGTAGCC ACAAACCAAA CTCGATACCT 1740
GCATATAGCG ATCTCGCTCC TCCTCCACTG CATCTATTTA CGAGACGGCG TTAGAACATT 1800
TCACGACATT CTGGCTTTAT TGCATCGAGC ACATTTCGAC ACATATATCT TTAATACCCT 1860
TTCTTCGGTG TCCCAGATCA TCGGTTCGAC CTTAATGTAC CTCGGTCCGA ATCCGCCTGG 1920
GATACTGTTT CTCTTTCCGC CGCACTTCAC TGTACATTGC TTGACATTGC GAAACCGGGT 1980
TGGGCTCGAA CGTGGGATGG GTTATCGCTC ATCGCTACAC GCCGTTGCTC CATCATAATG 2040
TTAATGGACA CAATGGGGCT ACGCATCCTG GTGTTTAGTC CTGGAAGACC ATCCGATAAC 2100
CCCCGTCGGT AACACTCGCT TGTCTCGTGT CCACCCAGAC ACTACTTCAA TTCTCACTTC 2160
TATCGTCCGC TATTACCTTG ACCTGGTCGA ACCCATCCTT ATTATTCGTT TCGACTATGC 2220
TATATATTTA TTTTTACCAT TCGTGTCGAT CGCTCATACT CTTGGCGCTT GGGACTGGAA 2280
GCATTTATAT TGGAAAAAAT CACGGAATGG GGCGCCTTTT CTTCTTGCAC TTCACTCGCT 2340
GTGCATAGAC GGTTTTACAT TTCTGCTTTG CAATGCATCA CGAACTCTGC ATTAGCATAT 2400
AGAAAGAGGG GAAGGATGGA CCTTCTTCTT GATTGCTCGC ATGGTTTATC CATTCGCTCA 2460
AAGTGGATTA CGTCCACATA TTACCCGGGG GCTATACACA TGGCTACTGT GTTGCTTTCT 2520
GACATTCGCC GGACGTGCAA GGTTGGGAGG AGAGTCTGAC GCTGACGGGG CTTGTTGAAG 2580
GATGTTCACG CGTCCCGATT TGACCCGGCT TCGACTAACC TCAGATTCTC GACTTGTTGG 2640
ACGGTGACTT GACTTGCTTG CTATGGTCTG ACGCTCTCAC ACCTACCTAT CACATCCTCC 2700
TCACCTCACA AATTCCGCTC ATGGACACTA TCCTCTTCTT TTCGTTTCCC TTGGATAGTG 2760
TGTGTGTGTG TGTGGTTGGG GCAAATTATC CATAGCAGCA GTATTATTAG TTATAATCCG 2820
GTAGTGTTAT GATTTATGAA GGCAACTTGT ATACTA 2856
SEQ ID NO : 4
   SEQUENCE LENGTH: 439
   SEQUENCE TYPE : amino acid
   STRANDEDNESS : single
   TOPOLOGY : linear
   MOLECULE TYPE : peptide
   SEQUENCE DESCRIPTION : 
Met Asn Gln Thr Leu Pro Thr Trp Lys Asp Arg Thr Gln Asn Gln
1               5                   10                  15
Phe Gly Lys Leu Gln Ile Gln Val Pro Trp Arg Ser Ile Gln Leu
                20                  25                  30
Leu Val Pro His Arg Met Arg Arg Lys Leu Arg Ser Lys Leu Arg
                35                  40                  45
Ser Arg Ala Ser Pro Thr Ser Ser Ile Ala Ser Leu Gln Thr Ser
                50                  55                  60
Leu Ser Pro Ala Asp Thr Leu Arg Ser Leu Gln Ser His Arg Trp
                65                  70                  75
Thr Val Tyr Asp Phe Gln Tyr Leu Leu Leu Leu Ile Val Gly Ile
                80                  85                  90
Phe Ser Leu Thr Val Ile Glu Ser Pro Gly Pro Leu Gly Lys Thr
                95                  100                 105
Ala Ile Phe Ser Met Leu Leu Phe Ser Leu Leu Ile Pro Met Thr
                110                 115                 120
Arg Gln Phe Phe Leu Pro Phe Leu Pro Ile Ala Gly Trp Leu Leu
                125                 130                 135
Phe Phe Tyr Ala Cys Gln Phe Ile Pro Ser Asp Trp Arg Pro Ala
                140                 145                 150
Ile Trp Val Arg Val Leu Pro Ala Leu Glu Asn Ile Leu Tyr Gly
                155                 160                 165
Ala Asn Ile Ser Asn Ile Leu Ser Ala His Gln Asn Val Val Leu
                170                 175                 180
 
Asp Val Leu Ala Trp Leu Pro Tyr Gly Ile Cys His Tyr Gly Ala
                185                 190                 195
Pro Phe Val Cys Ser Leu Ile Met Phe Ile Phe Gly Pro Pro Gly
                200                 205                 210
Thr Val Pro Leu Phe Ala Arg Thr Phe Gly Tyr Ile Ser Met Thr
                215                 220                 225
Ala Val Thr Ile Gln Leu Phe Phe Pro Cys Ser Pro Pro Trp Tyr
                230                 235                 240
Glu Asn Arg Tyr Gly Leu Ala Pro Ala Asp Tyr Ser Ile Gln Gly
                245                 250                 255
Asp Pro Ala Gly Leu Ala Arg Ile Asp Lys Leu Phe Gly Ile Asp
                260                 265                 270
Leu Tyr Thr Ser Gly Phe His Gln Ser Pro Val Val Phe Gly Ala
                275                 280                 285
Phe Pro Ser Leu His Ala Ala Asp Ser Thr Leu Ala Ala Leu Phe
                290                 295                 300
Met Ser His Val Phe Pro Arg Met Lys Pro Val Phe Val Thr Tyr
                305                 310                 315
Thr Leu Trp Met Trp Trp Ala Thr Met Tyr Leu Ser His His Tyr
                320                 325                 330
Ala Val Asp Leu Val Ala Gly Gly Leu Leu Ala Ala Ile Ala Phe
                335                 340                 345
Tyr Phe Ala Lys Thr Arg Phe Leu Pro Arg Val Gln Leu Asp Lys
                350                 355                 360
Thr Phe Arg Trp Asp Tyr Asp Tyr Val Glu Phe Gly Glu Ser Ala
                365                 370                 375
Leu Glu Tyr Gly Tyr Gly Ala Ala Gly Tyr Asp Gly Asp Phe Asn
                380                 385                 390
Leu Asp Ser Asp Glu Trp Thr Val Gly Ser Ser Ser Ser Val Ser
 
                395                 400                 405
Ser Gly Ser Leu Ser Pro Val Asp Asp His Tyr Ser Trp Glu Thr
                410                 415                 420
Glu Ala Leu Thr Ser Pro His Thr Asp Ile Glu Ser Gly Arg His
                425                 430                 435
Thr Phe Ser Pro
SEQ ID NO : 5
   SEQUENCE LENGTH: 436
   SEQUENCE TYPE: amino acid
   STRANDEDNESS : single
   TOPOLOGY: linear
   MOLECULE TYPE : peptide
   SEQUENCE DESCRIPTION : 
Met Asn Thr Thr Leu Pro Ser Trp Lys Asp Arg Thr Gln Asn Gln
1               5                   10                  15
Phe Gly Lys Leu Gln Ile Gln Val Pro Trp Arg Thr Ile Gln Leu
                20                  25                  30
Leu Val Pro His Arg Met Arg Arg Lys Ile Arg Ser Lys Leu Arg
                35                  40                  45
Ser Arg Ile Ser Pro Thr Ser Ser Ile Ser Ser Leu Gln Thr Ser
                50                  55                  60
Phe Ser Pro Val Asp Thr Leu Arg Ser Leu Gln Ser His Arg Trp
                65                  70                  75
Thr Leu Tyr Asp Phe Gln Tyr Leu Leu Leu Leu Ile Val Gly Ile
                80                  85                  90
Phe Ser Leu Ser Val Met Glu Ser Pro Gly Pro Leu Ala Lys Thr
                95                  100                 105
Ala Ala Phe Thr Leu Leu Leu Val Ser Leu Leu Leu Pro Ile Thr
 
                110                 115                 120
Arg Gln Phe Phe Leu Pro Phe Leu Pro Ile Ala Gly Trp Leu Ile
                125                 130                 135
Phe Phe Tyr Ala Cys Gln Phe Ile Pro Ser Asp Trp Arg Pro Ala
                140                 145                 150
Ile Trp Val Arg Val Leu Pro Ala Leu Glu Asn Ile Leu Tyr Gly
                155                 160                 165
Ala Asn Ile Ser Asn Ile Leu Ser Ala His Gln Asn Val Val Leu
                170                 175                 180
Asp Val Leu Ala Trp Leu Pro Tyr Gly Ile Cys His Tyr Gly Ala
                185                 190                 195
Pro Phe Val Cys Ser Ala Ile Met Phe Ile Phe Gly Pro Pro Gly
                200                 205                 210
Thr Val Pro Leu Phe Ala Arg Thr Phe Gly Tyr Ile Ser Met Ala
                215                 220                 225
Ala Val Thr Ile Gln Leu Phe Phe Pro Cys Ser Pro Pro Trp Tyr
                230                 235                 240
Glu Asn Leu Tyr Gly Leu Ala Pro Ala Asp Tyr Ser Met Pro Gly
                245                 250                 255
Asn Pro Ala Gly Leu Ala Arg Ile Asp Glu Leu Phe Gly Ile Asp
                260                 265                 270
Leu Tyr Thr Ser Gly Phe Arg Gln Ser Pro Val Val Phe Gly Ala
                275                 280                 285
Phe Pro Ser Leu His Ala Ala Asp Ser Thr Leu Ala Ala Leu Phe
                290                 295                 300
Met Ser Gln Val Phe Pro Arg Leu Lys Pro Leu Phe Val Ile Tyr
                305                 310                 315
Thr Leu Trp Met Trp Trp Ala Thr Met Tyr Leu Ser His His Tyr
                320                 325                 330
 
Ala Val Asp Leu Val Gly Gly Gly Leu Leu Ala Thr Val Ala Phe
                335                 340                 345
Tyr Phe Ala Lys Thr Arg Phe Met Pro Arg Val Gln Asn Asp Lys
                350                 355                 360
Met Phe Arg Trp Asp Tyr Asp Tyr Val Glu Tyr Gly Asp Ser Ala
                365                 370                 375
Leu Asp Tyr Gly Tyr Gly Pro Ala Ser Phe Glu Gly Glu Phe Asn
                380                 385                 390
Leu Asp Ser Asp Glu Trp Thr Val Gly Ser Ser Ser Ser Ile Ser
                395                 400                 405
Ser Gly Ser Leu Ser Pro Val Asp Asp His Tyr Ser Trp Glu Gly
                410                 415                 420
Glu Thr Leu Ala Ser Pro Ala Thr Asp Ile Glu Ser Gly Arg His
                425                 430                 435
Phe
SEQ ID NO : 6
   SEQUENCE LENGTH: 401
   SEQUENCE TYPE: amino acid
   STRANDEDNESS : single
   TOPOLOGY : linear
   MOLECULE TYPE: peptide
   SEQUENCE DESCRIPTION : 
Met Ala Asn Pro Phe Ser Arg Trp Phe Leu Ser Glu Arg Pro Pro
1               5                   10                  15
Asn Cys His Val Ala Asp Leu Glu Thr Ser Leu Asp Pro His Gln
                20                  25                  30
Thr Leu Leu Lys Val Gln Lys Tyr Lys Pro Ala Leu Ser Asp Trp
                35                  40                  45
 
Val His Tyr Ile Phe Leu Gly Ser Ile Met Leu Phe Val Phe Ile
                50                  55                  60
Thr Asn Pro Ala Pro Trp Ile Phe Lys Ile Leu Phe Tyr Cys Phe
                65                  70                  75
Leu Gly Thr Leu Phe Ile Ile Pro Ala Thr Ser Gln Phe Phe Phe
                80                  85                  90
Asn Ala Leu Pro Ile Leu Thr Trp Val Ala Leu Tyr Phe Thr Ser
                95                  100                 105
Ser Tyr Phe Pro Asp Asp Arg Arg Pro Pro Ile Thr Val Lys Val
                110                 115                 120
Leu Pro Ala Val Glu Thr Ile Leu Tyr Gly Asp Asn Leu Ser Asp
                125                 130                 135
Ile Leu Ala Thr Ser Thr Asn Ser Phe Leu Asp Ile Leu Ala Trp
                140                 145                 150
Leu Pro Tyr Gly Leu Phe His Phe Gly Ala Pro Phe Val Val Ala
                155                 160                 165
Ala Ile Leu Phe Val Phe Gly Pro Pro Thr Val Leu Gln Gly Tyr
                170                 175                 180
Ala Phe Ala Phe Gly Tyr Met Asn Leu Phe Gly Val Ile Met Gln
                185                 190                 195
Asn Val Phe Pro Ala Ala Pro Pro Trp Tyr Lys Ile Leu Tyr Gly
                200                 205                 210
Leu Gln Ser Ala Asn Tyr Asp Met His Gly Ser Pro Gly Gly Leu
                215                 220                 225
Ala Arg Ile Asp Lys Leu Leu Gly Ile Asn Met Tyr Thr Thr Ala
                230                 235                 240
Phe Ser Asn Ser Ser Val Ile Phe Gly Ala Phe Pro Ser Leu His
                245                 250                 255
Ser Gly Cys Ala Thr Met Glu Ala Leu Phe Phe Cys Tyr Cys Phe
 
                260                 265                 270
Pro Lys Leu Lys Pro Leu Phe Ile Ala Tyr Val Cys Trp Leu Trp
                275                 280                 285
Trp Ser Thr Met Tyr Leu Thr His His Tyr Phe Val Asp Leu Met
                290                 295                 300
Ala Gly Ser Val Leu Ser Tyr Val Ile Phe Gln Tyr Thr Lys Tyr
                305                 310                 315
Thr His Leu Pro Ile Val Asp Thr Ser Leu Phe Cys Arg Trp Ser
                320                 325                 330
Tyr Thr Ser Ile Glu Lys Tyr Asp Ile Ser Lys Ser Asp Pro Leu
                335                 340                 345
Ala Ala Asp Ser Asn Asp Ile Glu Ser Val Pro Leu Ser Asn Leu
                350                 355                 360
Glu Leu Asp Phe Asp Leu Asn Met Thr Asp Glu Pro Ser Val Ser
                365                 370                 375
Pro Ser Leu Phe Asp Gly Ser Thr Ser Val Ser Arg Ser Ser Ala
                380                 385                 390
Thr Ser Ile Thr Ser Leu Gly Val Lys Arg Ala
                395                 400
SEQ ID NO : 7
   SEQ LENGTH : 422
   SEQUENCE TYPE : amino acid
   STRANDEDNESS : single
   TOPOLOGY: linear
   MOLECULE TYPE: peptide
   SEQUENCE DESCRIPTION : 
Met Ser Ala Leu Ser Thr Leu Lys Lys Arg Leu Ala Ala Cys Asn
1               5                   10                  15
 
Arg Ala Ser Gln Tyr Lys Leu Glu Thr Ser Leu Asn Pro Met Pro
                20                  25                  30
Thr Phe Arg Leu Leu Arg Asn Thr Lys Trp Ser Trp Thr His Leu
                35                  40                  45
Gln Tyr Val Phe Leu Ala Gly Asn Leu Ile Phe Ala Cys Ile Val
                50                  55                  60
Ile Glu Ser Pro Gly Phe Trp Gly Lys Phe Gly Ile Ala Cys Leu
                65                  70                  75
Leu Ala Ile Ala Leu Thr Val Pro Leu Thr Arg Gln Ile Phe Phe
                80                  85                  90
Pro Ala Ile Val Ile Ile Thr Trp Ala Ile Leu Phe Tyr Ser Cys
                95                  100                 105
Arg Phe Ile Pro Glu Arg Trp Arg Pro Pro Ile Trp Val Arg Val
                110                 115                 120
Leu Pro Thr Leu Glu Asn Ile Leu Tyr Gly Ser Asn Leu Ser Ser
                125                 130                 135
Leu Leu Ser Lys Thr Thr His Ser Ile Leu Asp Ile Leu Ala Trp
                140                 145                 150
Val Pro Tyr Gly Val Met His Tyr Ser Ala Pro Phe Ile Ile Ser
                155                 160                 165
Phe Ile Leu Phe Ile Phe Ala Pro Pro Gly Thr Leu Pro Val Trp
                170                 175                 180
Ala Arg Thr Phe Gly Tyr Met Asn Leu Phe Gly Val Leu Ile Gln
                185                 190                 195
Met Ala Phe Pro Cys Ser Pro Pro Trp Tyr Glu Asn Met Tyr Gly
                200                 205                 210
Leu Glu Pro Ala Thr Tyr Ala Val Arg Gly Ser Pro Gly Gly Leu
                215                 220                 225
Ala Arg Ile Asp Ala Leu Phe Gly Thr Ser Ile Tyr Thr Asp Gly
 
                230                 235                 240
Phe Ser Asn Ser Pro Val Val Phe Gly Ala Phe Pro Ser Leu His
                245                 250                 255
Ala Gly Trp Ala Met Leu Glu Ala Leu Phe Leu Ser His Val Phe
                260                 265                 270
Pro Arg Tyr Arg Phe Cys Phe Tyr Gly Tyr Val Leu Trp Leu Cys
                275                 280                 285
Trp Cys Thr Met Tyr Leu Thr His His Tyr Phe Val Asp Leu Val
                290                 295                 300
Gly Gly Met Cys Leu Ala Ile Ile Cys Phe Val Phe Ala Gln Lys
                305                 310                 315
Leu Arg Leu Pro Gln Leu Gln Thr Gly Lys Ile Leu Arg Trp Glu
                320                 325                 330
Tyr Glu Phe Val Ile His Gly His Gly Leu Ser Glu Lys Thr Ser
                335                 340                 345
Asn Ser Leu Ala Arg Thr Gly Ser Pro Tyr Leu Leu Gly Arg Asp
                350                 355                 360
Ser Phe Thr Gln Asn Pro Asn Ala Val Ala Phe Met Ser Gly Leu
                365                 370                 375
Asn Asn Met Glu Leu Ala Asn Thr Asp His Glu Trp Ser Val Gly
                380                 385                 390
Ser Ser Ser Pro Glu Pro Leu Pro Ser Pro Ala Ala Asp Leu Ile
                395                 400                 405
Asp Arg Pro Ala Ser Thr Thr Ser Ser Ile Phe Asp Ala Ser His
                410                 415                 420
Leu Pro
SEQ ID NO : 8
   SEQUENCE LENGTH: 471
   SEQUENCE TYPE: amino acid
   STRANDEDNESS : single
   TOPOLOGY: linear
   MOLECULE TYPE : peptide
   SEQUENCE DESCRIPTION : 
Met Ala Ser Ser Ile Leu Arg Ser Lys Ile Ile Gln Lys Pro Tyr
1               5                   10                  15
Gln Leu Phe His Tyr Tyr Phe Leu Ser Glu Lys Ala Pro Gly Ser
                20                  25                  30
Thr Val Ser Asp Leu Asn Phe Asp Thr Asn Ile Gln Thr Ser Leu
                35                  40                  45
Arg Lys Leu Lys His His His Trp Thr Val Gly Glu Ile Phe His
                50                  55                  60
Tyr Gly Phe Leu Val Ser Ile Leu Phe Phe Val Phe Val Val Phe
                65                  70                  75
Pro Ala Ser Phe Phe Ile Lys Leu Pro Ile Ile Leu Ala Phe Ala
                80                  85                  90
Thr Cys Phe Leu Ile Pro Leu Thr Ser Gln Phe Phe Leu Pro Ala
                95                  100                 105
Leu Pro Val Phe Thr Trp Leu Ala Leu Tyr Phe Thr Cys Ala Lys
                110                 115                 120
Ile Pro Gln Glu Trp Lys Pro Ala Ile Thr Val Lys Val Leu Pro
                125                 130                 135
Ala Met Glu Thr Ile Leu Tyr Gly Asp Asn Leu Ser Asn Val Leu
                140                 145                 150
Ala Thr Ile Thr Thr Gly Val Leu Asp Ile Leu Ala Trp Leu Pro
                155                 160                 165
Tyr Gly Ile Ile His Phe Ser Phe Pro Phe Val Leu Ala Ala Ile
                170                 175                 180
 
Ile Phe Leu Phe Gly Pro Pro Thr Ala Leu Arg Ser Phe Gly Phe
                185                 190                 195
Ala Phe Gly Tyr Met Asn Leu Leu Gly Val Leu Ile Gln Met Ala
                200                 205                 210
Phe Pro Ala Ala Pro Pro Trp Tyr Lys Asn Leu His Gly Leu Glu
                215                 220                 225
Pro Ala Asn Tyr Ser Met His Gly Ser Pro Gly Gly Leu Gly Arg
                230                 235                 240
Ile Asp Lys Leu Leu Gly Val Asp Met Tyr Thr Thr Gly Phe Ser
                245                 250                 255
Asn Ser Ser Ile Ile Phe Gly Ala Phe Pro Ser Leu His Ser Gly
                260                 265                 270
Cys Cys Ile Met Glu Val Leu Phe Leu Cys Trp Leu Phe Pro Arg
                275                 280                 285
Phe Lys Phe Val Trp Val Thr Tyr Ala Ser Trp Leu Trp Trp Ser
                290                 295                 300
Thr Met Tyr Leu Thr His His Tyr Phe Val Asp Leu Ile Gly Gly
                305                 310                 315
Ala Met Leu Ser Leu Thr Val Phe Glu Phe Thr Lys Tyr Lys Tyr
                320                 325                 330
Leu Pro Lys Asn Lys Glu Gly Leu Phe Cys Arg Trp Ser Tyr Thr
                335                 340                 345
Glu Ile Glu Lys Ile Asp Ile Gln Glu Ile Asp Pro Leu Ser Tyr
                350                 355                 360
Asn Tyr Ile Pro Val Asn Ser Asn Asp Asn Glu Ser Arg Leu Tyr
                365                 370                 375
Thr Arg Val Tyr Gln Glu Ser Gln Val Ser Pro Pro Gln Arg Ala
                380                 385                 390
Glu Thr Pro Glu Ala Phe Glu Met Ser Asn Phe Ser Arg Ser Arg
 
                395                 400                 405
Gln Ser Ser Lys Thr Gln Val Pro Leu Ser Asn Leu Thr Asn Asn
                410                 415                 420
Asp Gln Val Ser Gly Ile Asn Glu Glu Asp Glu Glu Glu Glu Gly
                425                 430                 435
Asp Glu Ile Ser Ser Ser Thr Pro Ser Val Phe Glu Asp Glu Pro
                440                 445                 450
Gln Gly Ser Thr Tyr Ala Ala Ser Ser Ala Thr Ser Val Asp Asp
                455                 460                 465
Leu Asp Ser Lys Arg Asn
                470
SEQ ID NO : 9
   SEQUENCE LENGTH : 10
   SEQUENCE TYPE: amino acid
   STRANDEDNESS : single
   TOPOLOGY: linear
   MOLECULE TYPE : peptide
   FEATURE : Xaa at position 3 is Val or Ile.
      Xaa at position 7 is Leu or Val.
   SEQUENCE DESCRIPTION : 
Leu Asp Xaa Leu Ala Trp Xaa Pro Tyr Gly
1               5                   10
SEQ ID NO : 10
   SEQUENCE LENGTH : 8
   SEQUENCE TYPE : amino acid
   STRANDEDNESS : single
   TOPOLOGY: linear
   MOLECULE TYPE: peptide
   SEQUENCE DESCRIPTION : 
Phe Gly Ala Phe Pro Ser Leu His
1               5
SEQ ID NO : 11
   SEQUENCE LENGTH: 12
   SEQUENCE TYPE: amino acid
   STRANDEDNESS : single
   TOPOLOGY: linear
   MOLECULE TYPE: peptide
   FEATURE : Xaa at position 5 is Ser or Thr.
      Xaa at position 9 is Ala or Phe.
   SEQUENCE DESCRIPTION : 
Thr Met Tyr Leu Xaa His His Tyr Xaa Val Asp Leu
1               5                   10
SEQ ID NO : 12
   SEQUENCE LENGTH: 2935
   SEQUENCE TYPE: nucleic acid
   STRANDEDNESS : double
   TOPOLOGY: linear
   MOLECULE TYPE: cDNA
   SEQUENCE DESCRIPTION : 
ATTTTCTTCC CCATAACAAC TCTTCTCGCC CTTCCTCCGG CTCCGTGGCC AAATTGTTTT 60
ATGCAGCGCC TCCTAGCGAT TTAACCTCGT TCTCGTTGCC CTTGCCTGTC CGCCTTGCGT 120
CAGTACGACC CTTGCAACGT GACCTTCCCC AGAGTATCCT CGTTTGGCCG CTGGAGACCG 180
GAGCTTGCAC CCTCATAAAC TAGCTCTTCG AAATCAATTC TCCGTTCTCC AGAGATTATC 240
GGATCGAATC TCTCCGCTGT CGACACCTTT CGTCTCTCGG TGATCCTCGC CCTTGGAGTC 300
 
TCGTCACGTT GACGCCTTGA ACCCCTGGCC GCCAACTCCA CATAGGAGAC CACACTTCAT 360
TCTTCCCCCG CCATAATTGC AGCACCCTCC GTCTCCCTTC GAGCTCCTCC TGGATCATCA 420
AGTCCGAAAG GATTAGACTC GTCGCAGCGA TGAATACCAC CCTTCCATCC TGGAAGGATC 480
GGACGCAAAA CCAGTTCGGC AAGCTCCAGA TCCAAGTCCC ATGGCGCACC ATACAACTTC 540
TCGTGCCGCA CCGTATGCGA CGGAAGATTC GGTCCAAGCT GCGCAGTCGG ATCTCGCCTA 600
CCTCATCGAT ATCCTCGTTG CAGACGTCAT TCTCACCTGT CGATACACTC AGGTCGCTGC 660
AAAGTCATAG ATGGACGCTC TATGACTTTC AGTATCTTTT GCTGCTGATT GTCGGCATAT 720
TCTCGCTGAG CGTTATGGAA TCACCTGGAC CATTGGCAAA GACCGCCGCG TTTACGCTAC 780
TTCTCGTCTC TCTCCTTCTC CCGATTACGC GCCAGTTCTT CTTGCCATTC CTCCCGATTG 840
CAGGATGGCT TATATTTTTC TACGCTTGCC AGTTCATCCC GAGCGACTGG CGCCCTGCAA 900
TCTGGGTTCG CGTGCTGCCG GCTCTGGAAA ACATTCTCTA CGGTGCTAAT ATCAGTAACA 960
TCCTTTCCGC TCACCAAAAT GTGGTGCTTG ACGTTTTGGC GTGGCTTCCC TACGGAATCT 1020
GCCATTATGG CGCGCCATTT GTGTGCTCAG CGATCATGTT CATCTTTGGT CCTCCCGGCA 1080
CCGTCCCCCT TTTCGCTCGA ACTTTTGGAT ACATCAGCAT GGCTGCAGTC ACCATTCAGC 1140
TGTTTTTCCC CTGCTCTCCT CCGTGGTACG AAAATCTGTA TGGTTTGGCT CCGGCTGATT 1200
ACTCCATGCC GGGTAATCCT GCGGGCCTTG CTCGCATCGA TGAGCTTTTT GGGATAGACT 1260
TGTACACATC GGGCTTCAGA CAATCTCCCG TCGTGTTTGG CGCATTTCCT TCCCTACATG 1320
CCGCTGATTC GACACTTGCA GCTCTATTTA TGAGCCAAGT GTTCCCACGG TTGAAGCCCT 1380
TGTTTGTCAT CTATACTCTC TGGATGTGGT GGGCTACAAT GTATCTTTCG CACCACTACG 1440
CTGTTGATCT GGTCGGTGGT GGCCTCTTGG CAACTGTCGC GTTCTACTTT GCTAAAACGC 1500
GGTTCATGCC TCGCGTCCAG AATGATAAGA TGTTCCGCTG GGACTACGAT TATGTTGAGT 1560
ACGGCGATTC CGCACTCGAC TATGGGTACG GTCCAGCCAG CTTCGAAGGC GAATTCAACC 1620
TTGATAGCGA TGAGTGGACC GTTGGTTCTT CGTCATCCAT TTCGTCCGGC TCCCTCAGTC 1680
CAGTTGACGA CCACTACTCT TGGGAGGGCG AGACTCTTGC CTCTCCTGCC ACCGACATCG 1740
AGTCTGGAAG GCATTTCTGA TCCTGCTCAA TGAGCCTTGA TACGTACTAC ACTGTGTACG 1800
TGCTACTGCA TTGACTAATG AGACGGCGTT TTCAAACAAA TTTTAACGAC ATGCTTGGTT 1860
ATCGCATTGA GCTGATTTCG ACACATATAT ATGTTTAATA CGTTTTGGGG ACACTCCAGG 1920
GATTCATGAC GGTTGCTTCA TATCCCGACC TGGGGATGGA TTGACCTGGT TGTGCCCAAT 1980
TTTCTTCTGC CTAACGTTTT GATTATACAT GCATTTTTCA CGAAACCAGC CGGCCCGCCA 2040
 
TGATCGTGAC CTCAATTTGA GCTCGAATCT TCCTGGGGCC TCCAGCGATA ATTCTTAATG 2100
CTCGTTCCGA GGGTGCCACA TCGGACATTC GCTTGTACAA CTTTTGCAGA ACGAACATTT 2160
TCACCGATTC CAACTTGAGT CATTCGCTTA CTACTTTCAA CTGGTCGAGA AACTTCGCTT 2220
CTTTTCAGCT CGGCTAGGTG CATAAATATT TTACATTCGT GTCGATCGCT CACATTTCAC 2280
GGCGCCTGGA AACTTGGGGG TTTCGATTTC ATTGGAAAGG ATAAACAACA TGGGCTGGGC 2340
GCCTTTTACA CGCACTACAT TCGCTTAGAA AAGTTCTGAT GCTTTTAATG ATTCTTGCAT 2400
TGGCATATAG AAAGGGGTCC TCCAGACTCG CTACTGTGGT CCTCTCTCAA CCCCACACTC 2460
GCTTGCTTTT AACAGTGGAC ACCCCGTGGA GCTACGTCTC CATCAAATAT TTGGCATCAA 2520
CCGGAATCGA TGCCAGGAGG ACTGAGCTTA CTCACGGTGA CCGTCGGGTA AAAGGCGTTC 2580
ATACAGAACA TCTCCTCTAT CCTCCTGTCC CATCTCGATT CTCTGGCTGC TGGACGCAAC 2640
ACACCTCGCT GCACGTTTTC GACTTCCTAA TACGACCTAA TATCATTCTC GGTTTTCTTT 2700
GCTCTGGCTC GCGGCCGCCA TTTATATGGC GTGTCGGCTC GAGTCTGAAG TGAACTTTTT 2760
TCTCCTTTCT GGCCTCCACA ACTTTCCGAT CCCTAGCAGC TTCCCGTGCA CAGCGAGGTG 2820
TTGTTGGATG ATTGTTCCAT AGCATTATCA TTATTCCTAA TCCGGTAGCG TTATGATTTA 2880
TGAAGAACAG TGATGTACAT TATTATGCGG TGATCAAAAA AAAAAAAAAA AAAAA 2935
SEQ I D NO : 13
   SEQUENCE LENGTH : 2856
   SEQUENCE TYPE : nucleic acid
   STRANDEDNESS : double
   TOPOLOGY: linear
   MOLECULE TYPE: Genomic DNA
   ANTI-SENSE : yes
   SEQUENCE DESCRIPTION : 
TAGTATACAA GTTGCCTTCA TAAATCATAA CACTACCGGA TTATAACTAA TAATACTGCT 60
GCTATGGATA ATTTGCCCCA ACCACACACA CACACACACT ATCCAAGGGA AACGAAAAGA 120
AGAGGATAGT GTCCATGAGC GGAATTTGTG AGGTGAGGAG GATGTGATAG GTAGGTGTGA 180
GAGCGTCAGA CCATAGCAAG CAAGTCAAGT CACCGTCCAA CAAGTCGAGA ATCTGAGGTT 240
AGTCGAAGCC GGGTCAAATC GGGACGCGTG AACATCCTTC AACAAGCCCC GTCAGCGTCA 300
 
GACTCTCCTC CCAACCTTGC ACGTCCGGCG AATGTCAGAA AGCAACACAG TAGCCATGTG 360
TATAGCCCCC GGGTAATATG TGGACGTAAT CCACTTTGAG CGAATGGATA AACCATGCGA 420
GCAATCAAGA AGAAGGTCCA TCCTTCCCCT CTTTCTATAT GCTAATGCAG AGTTCGTGAT 480
GCATTGCAAA GCAGAAATGT AAAACCGTCT ATGCACAGCG AGTGAAGTGC AAGAAGAAAA 540
GGCGCCCCAT TCCGTGATTT TTTCCAATAT AAATGCTTCC AGTCCCAAGC GCCAAGAGTA 600
TGAGCGATCG ACACGAATGG TAAAAATAAA TATATAGCAT AGTCGAAACG AATAATAAGG 660
ATGGGTTCGA CCAGGTCAAG GTAATAGCGG ACGATAGAAG TGAGAATTGA AGTAGTGTCT 720
GGGTGGACAC GAGACAAGCG AGTGTTACCG ACGGGGGTTA TCGGATGGTC TTCCAGGACT 780
AAACACCAGG ATGCGTAGCC CCATTGTGTC CATTAACATT ATGATGGAGC AACGGCGTGT 840
AGCGATGAGC GATAACCCAT CCCACGTTCG AGCCCAACCC GGTTTCGCAA TGTCAAGCAA 900
TGTACAGTGA AGTGCGGCGG AAAGAGAAAC AGTATCCCAG GCGGATTCGG ACCGAGGTAC 960
ATTAAGGTCG AACCGATGAT CTGGGACACC GAAGAAAGGG TATTAAAGAT ATATGTGTCG 1020
AAATGTGCTC GATGCAATAA AGCCAGAATG TCGTGAAATG TTCTAACGCC GTCTCGTAAA 1080
TAGATGCAGT GGAGGAGGAG CGAGATCGCT ATATGCAGGT ATCGAGTTTG GTTTGTGGCT 1140
ACTCAAGGGC TGAAAGTATG CCTGCCGGAC TCAATATCAG TATGTGGGGA GGTCAGTGCC 1200
TCGGTTTCCC ATGAGTAATG ATCGTCAACG GGACTCAAGG AGCCTGAGGA GACGGAGGAT 1260
GAAGAACCAA CAGTCCATTC ATCGCTGTCG AGATTGAAGT CTCCATCATA GCCAGCTGCA 1320
CCATACCCAT ACTCCAGGGC AGACTCGCCG AATTCCACAT AGTCGTAGTC CCAACGGAAG 1380
GTCTTGTCGA GCTGGACACG GGGAAGGAAT CGGGTCTTGG CGAAGTAGAA AGCAATGGCG 1440
GCCAGGAGAC CACCCGCAAC CAAATCGACC GCATAGTGAT GTGAGAGGTA CATTGTTGCC 1500
CACCACATCC ATAGAGTATA GGTCACGAAG ACGGGCTTCA TGCGGGGGAA AACATGACTC 1560
ATGAAAAGTG CGGCCAGGGT TGAGTCGGCA GCATGCAGCG ACGGAAAAGC GCCGAACACA 1620
ACAGGCGACT GATGGAAAAC AGACGTGTAA AGGTCGATGC CGAAAAGCTT GTCAATGCGG 1680
GCAAGCCCTG CGGGATCACC TTGGATGGAG TAGTCTGCCG GAGCTAGACC ATAGCGATTC 1740
TCATACCAAG GTGGAGAGCA AGGGAAAAAC AGCTGAATAG TAACCGCAGT CATACTGATA 1800
TAGCCGAAAG TGCGCGCGAA AAGGGGAACA GTGCCGGGCG GACCGAAGAT GAACATGATC 1860
AACGAGCACA CAAACGGAGC GCCATAGTGG CAGATACCGT AGGGTAGCCA CGCCAGCACG 1920
TCAAGCACAA CGTTCTGGTG AGCGGATAGG ATGTTGCTGA TGTTTGCGCC GTAGAGAATA 1980
TTCTCCAGTG CAGGCAAGAC ACGAACCCAA ATCGCAGGGC GCCAATCGCT TGGGATGAAC 2040
 
TGGCAGGCGT AGAAAAACAG AAGCCATCCG GCAATCGGCA GAAACGGGAG GAAGAACTGG 2100

CGGGTCATAG GGATCAGGAG AGAGAATAGG AGCATGGAGA AAATGGCCGT TTTGCCCAAA 2160

GGCCCGGGCG ACTCGATAAC GGTCAAAGAG AAGATGCCCA CGATCAACAG AAGCAGATAT 2220

TGGAAGTCGT AAACCGTCCA TCGGTGGCTT TGGAGCGATC GTAGTGTGTC TGCAGGCGAT 2280

AACGACGTCT GTAAAGAGGC TATTGACGAG GTAGGAGACG CTCTACTGCG CAATTTGGAC 2340

CTTAACTTCC GCCGCATGCG ATGCGGGACG AGCAGTTGGA TGGACCGCCA TGGAACCTGG 2400

ATCTGAAGCT TTCCAAACTG GTTCTGCGTG CGGTCCTTCC ACGTGGGAAG TGTTTGATTC 2460

ATGGTTGCGG CGAGTCTTGC GGGCAAGACT CAGCTATAAT TCCTCCAGAT CGAACTCCGT 2520

CTGCTTTCTT TCCCCCCGAT TGTACAAGAG TTGGAAGGCG ACGAATAAAT CGGTCGCGGA 2580

AGCGGCAGAA AAGGGGTCTT GAATAACAAG CCGTTAGGCA ACGAGCAGGG TGCTGACGGC 2640

GTCAACCCGA GCGGGGAAAT TGAGATCGGT TCGAAGAAGA CGAAACGAAG CGCACGCTCC 2700

GGTCTCCAGC GGCCAAACGA GGATGTCTTT GAGCCGTCAC GTTGCAGGGG GTTTCACTGA 2760

TGCAAGGCGA AGTGCAAGGG CGCAGCAGTC GAGAGGAAAA CCTGGAACGA GGAGGTCGTG 2820

AGGGAGGCAG ATGGCCACGG AGCCGGAGTA TAAACC 2856
SEQ ID NO: 14
   SEQUENCE LENGTH : 2856
   SEQUENCE TYPE: nucleic acid
   STRANDEDNESS : single
   TOPOLOGY: linear
   MOLECULE TYPE: mRNA
   ANTI-SENSE : yes
   SEQUENCE DESCRIPTION :

UAGUAUACAA GUUGCCUUCA UAAAUCAUAA CACUACCGGA UUAUAACUAA UAAUACUGCU 60

GCUAUGGAUA AUUUGCCCCA ACCACACACA CACACACACU AUCCAAGGGA AACGAAAAGA 120

AGAGGAUAGU GUCCAUGAGC GGAAUUUGUG AGGUGAGGAG GAUGUGAUAG GUAGGUGUGA 180

GAGCGUCAGA CCAUAGCAAG CAAGUCAAGU CACCGUCCAA CAAGUCGAGA AUCUGAGGUU 240

AGUCGAAGCC GGGUCAAAUC GGGACGCGUG AACAUCCUUC AACAAGCCCC GUCAGCGUCA 300

GACUCUCCUC CCAACCUUGC ACGUCCGGCG AAUGUCAGAA AGCAACACAG UAGCCAUGUG 360

UAUAGCCCCC GGGUAAUAUG UGGACGUAAU CCACUUUGAG CGAAUGGAUA AACCAUGCGA 420
GCAAUCAAGA AGAAGGUCCA UCCUUCCCCU CUUUCUAUAU GCUAAUGCAG AGUUCGUGAU 480
GCAUUGCAAA GCAGAAAUGU AAAACCGUCU AUGCACAGCG AGUGAAGUGC AAGAAGAAAA 540
GGCGCCCCAU UCCGUGAUUU UUUCCAAUAU AAAUGCUUCC AGUCCCAAGC GCCAAGAGUA 600
UGAGCGAUCG ACACGAAUGG UAAAAAUAAA UAUAUAGCAU AGUCGAAACG AAUAAUAAGG 660
AUGGGUUCGA CCAGGUCAAG GUAAUAGCGG ACGAUAGAAG UGAGAAUUGA AGUAGUGUCU 720
GGGUGGACAC GAGACAAGCG AGUGUUACCG ACGGGGGUUA UCGGAUGGUC UUCCAGGACU 780
AAACACCAGG AUGCGUAGCC CCAUUGUGUC CAUUAACAUU AUGAUGGAGC AACGGCGUGU 840
AGCGAUGAGC GAUAACCCAU CCCACGUUCG AGCCCAACCC GGUUUCGCAA UGUCAAGCAA 900
UGUACAGUGA AGUGCGGCGG AAAGAGAAAC AGUAUCCCAG GCGGAUUCGG ACCGAGGUAC 960
AUUAAGGUCG AACCGAUGAU CUGGGACACC GAAGAAAGGG UAUUAAAGAU AUAUGUGUCG 1020
AAAUGUGCUC GAUGCAAUAA AGCCAGAAUG UCGUGAAAUG UUCUAACGCC GUCUCGUAAA 1080
UAGAUGCAGU GGAGGAGGAG CGAGAUCGCU AUAUGCAGGU AUCGAGUUUG GUUUGUGGCU 1140
ACUCAAGGGC UGAAAGUAUG CCUGCCGGAC UCAAUAUCAG UAUGUGGGGA GGUCAGUGCC 1200
UCGGUUUCCC AUGAGUAAUG AUCGUCAACG GGACUCAAGG AGCCUGAGGA GACGGAGGAU 1260
GAAGAACCAA CAGUCCAUUC AUCGCUGUCG AGAUUGAAGU CUCCAUCAUA GCCAGCUGCA 1320
CCAUACCCAU ACUCCAGGGC AGACUCGCCG AAUUCCACAU AGUCGUAGUC CCAACGGAAG 1380
GUCUUGUCGA GCUGGACACG GGGAAGGAAU CGGGUCUUGG CGAAGUAGAA AGCAAUGGCG 1440
GCCAGGAGAC CACCCGCAAC CAAAUCGACC GCAUAGUGAU GUGAGAGGUA CAUUGUUGCC 1500
CACCACAUCC AUAGAGUAUA GGUCACGAAG ACGGGCUUCA UGCGGGGGAA AACAUGACUC 1560
AUGAAAAGUG CGGCCAGGGU UGAGUCGGCA GCAUGCAGCG ACGGAAAAGC GCCGAACACA 1620
ACAGGCGACU GAUGGAAAAC AGACGUGUAA AGGUCGAUGC CGAAAAGCUU GUCAAUGCGG 1680
GCAAGCCCUG CGGGAUCACC UUGGAUGGAG UAGUCUGCCG GAGCUAGACC AUAGCGAUUC 1740
UCAUACCAAG GUGGAGAGCA AGGGAAAAAC AGCUGAAUAG UAACCGCAGU CAUACUGAUA 1800
UAGCCGAAAG UGCGCGCGAA AAGGGGAACA GUGCCGGGCG GACCGAAGAU GAACAUGAUC 1860
AACGAGCACA CAAACGGAGC GCCAUAGUGG CAGAUACCGU AGGGUAGCCA CGCCAGCACG 1920
UCAAGCACAA CGUUCUGGUG AGCGGAUAGG AUGUUGCUGA UGUUUGCGCC GUAGAGAAUA 1980
UUCUCCAGUG CAGGCAAGAC ACGAACCCAA AUCGCAGGGC GCCAAUCGCU UGGGAUGAAC 2040
UGGCAGGCGU AGAAAAACAG AAGCCAUCCG GCAAUCGGCA GAAACGGGAG GAAGAACUGG 2100

CGGGUCAUAG GGAUCAGGAG AGAGAAUAGG AGCAUGGAGA AAAUGGCCGU UUUGCCCAAA 2160
GGCCCGGGCG ACUCGAUAAC GGUCAAAGAG AAGAUGCCCA CGAUCAACAG AAGCAGAUAU 2220
UGGAAGUCGU AAACCGUCCA UCGGUGGCUU UGGAGCGAUC GUAGUGUGUC UGCAGGCGAU 2280
AACGACGUCU GUAAAGAGGC UAUUGACGAG GUAGGAGACG CUCUACUGCG CAAUUUGGAC 2340
CUUAACUUCC GCCGCAUGCG AUGCGGGACG AGCAGUUGGA UGGACCGCCA UGGAACCUGG 2400
AUCUGAAGCU UUCCAAACUG GUUCUGCGUG CGGUCCUUCC ACGUGGGAAG UGUUUGAUUC 2460
AUGGUUGCGG CGAGUCUUGC GGGCAAGACU CAGCUAUAAU UCCUCCAGAU CGAACUCCGU 2520
CUGCUUUCUU UCCCCCCGAU UGUACAAGAG UUGGAAGGCG ACGAAUAAAU CGGUCGCGGA 2580
AGCGGCAGAA AAGGGGUCUU GAAUAACAAG CCGUUAGGCA ACGAGCAGGG UGCUGACGGC 2640
GUCAACCCGA GCGGGGAAAU UGAGAUCGGU UCGAAGAAGA CGAAACGAAG CGCACGCUCC 2700
GGUCUCCAGC GGCCAAACGA GGAUGUCUUU GAGCCGUCAC GUUGCAGGGG GUUUCACUGA 2760
UGCAAGGCGA AGUGCAAGGG CGCAGCAGUC GAGAGGAAAA CCUGGAACGA GGAGGUCGUG 2820
AGGGAGGCAG AUGGCCACGG AGCCGGAGUA UAAACC 2856

## Claims

1. A gene regulating aureobasidin sensitivity which is selected from the group consisting of:
(1) a gene encoding an amino acid sequence represented by SEQ. ID. No. 2, 4 or 5;
(2) a gene having a base sequence represented by SEQ. ID. No. 1, 3 or 12;
(3) a gene which hybridizes with a gene of (2) under a hybridisation condition wherein a hybridisation is effected in a solution containing 6 x SSC, 1% of sodium lauryl sulfate, 100 µ g/ml of salmon sperm DNA and 5 x Denhardt's solution (containing bovine serum albumin, polyvinylpyrrolidone and Ficol each at a concentration of 0.1%) at 65°C for 20 hours.

2. A gene as claimed in claim 1 which is obtained from a mold belonging to the genus *Aspergillus*.

3. A gene according to claim 1 or 2 encoding a protein capable of imparting resistance to aureobasidin.

4. A gene according to claim 3 represented by SEQ. ID. No. 1 in the Sequence Listing.

5. The antisense DNA of a gene according to claim 4 represented by SEQ. ID. No. 13 in the Sequence Listing.

6. The antisense RNA of a gene according to claim 4 represented by SEQ. ID. No. 14 in the Sequence Listing.

7. A recombinant plasmid containing a gene as claimed in any preceding claim.

8. A transformant having a recombinant plasmid as claimed in claim 7 introduced thereinto.

9. A protein regulating aureobasidin sensitivity encoded by a gene as claimed in claim 1 or 2.

10. A protein capable of imparting the resistance to aureobasidin, wherein at least the amino acid Gly at the position 275 of the protein imparting aureobasidin sensitivity represented by SEQ. ID. No. 4 in the Sequence Listing has been replaced by another amino acid.

11. A protein capable of imparting the resistance to aureobasidin as claimed in claim 10, wherein the amino acid Gly at the position 275 has been replaced by Val.

12. A process for producing the protein regulating aureobasidin sensitivity of claims 9, 10 or 11 which comprises incubating a transformant as claimed in claim 8 and recovering the protein.

## Patentansprüche

1. Aureobasidinempfindlichkeit regulierendes Gen, das ausgewählt ist aus der Gruppe bestehend aus:
(1) einem Gen, das für eine Aminosäuresequenz kodiert, wie sie durch SEQ. ID Nr. 2, 4 oder 5 dargestellt ist;
(2) einem Gen, das eine Basensequenz aufweist, wie sie durch SEQ ID Nr. 1, 3 oder 12 dargestellt ist;
(3) einem Gen, das mit einem Gen von (2) unter Hybridisierungsbedingungen hybridisiert, worin eine Hybridisierung in einer Lösung, die 6 x SSC, 1 % Natriumlaurylsulfat, 100 µg/ml Lachssperm-DNA und 5 x Denhardt-Lösung (die Rinderserumalbumin, Polyvinylpyrrolidon und Ficol jeweils in einer Konzentration von 0,1 % enthält) enthält, bei 65 °C über 20 Stunden vorgenommen wird.

2. Gen nach Anspruch 1, das von einem Pilz erhalten wird, der zur Gattung Aspergillus gehört.

3. Gen nach Anspruch 1 oder 2, das für ein Protein kodiert, das in der Lage ist, Resistenz gegen Aureobasidin zu verleihen.

4. Gen nach Anspruch 3, das durch SEQ ID Nr. 1 in der Sequenzliste dargestellt ist.

5. Antisense-DNA eines Gens nach Anspruch 4, dargestellt durch SEQ ID Nr. 13 in der Sequenzliste.

6. Antisense-RNA eines Gens nach Anspruch 4, dargestellt durch SEQ ID Nr. 14 in der Sequenzliste.

7. Rekombinantes Plasmid, das ein Gen enthält, wie es in einem der vorhergehenden Ansprüche beansprucht ist.

8. Transformante, bei der ein rekombinantes Plasmid, wie in Anspruch 7 beansprucht, eingeführt ist.

9. Protein, das Aureobasidinempfindlichkeit reguliert, kodiert durch ein Gen wie in Anspruch 1 oder 2 beansprucht.

10. Protein, das in der Lage ist, Resistenz gegen Aureobasidin zu verleihen, worin mindestens die Aminosäure Gly an der Position 275 des Proteins, das in der Lage ist, Resistenz gegen Aureobasidin zu verleihen, dargestellt durch SEQ ID Nr. 4 in der Sequenzliste, durch eine andere Aminosäure ersetzt ist.

11. Protein, das in der Lage ist, Resistenz gegen Aureobasidin zu verleihen wie in Anspruch 10 beansprucht, worin die Aminosäure Gly an der Position 275 durch Val ersetzt ist.

12. Verfahren zum Herstellen des Aureobasidinempfindlichkeit regulierenden Proteins nach Anspruch 9, 10 oder 11, das umfasst: Inkubieren einer Transformante wie in Anspruch 8 beansprucht und Gewinnen des Proteins.

## Revendications

1. Gène régulant la sensibilité à l'auréobasidine, qui est choisi dans le groupe consistant en :
(1) un gène codant pour une séquence d'aminoacides représentée par la SEQ N° 2, 4 ou 5 ;
(2) un gène ayant une séquence de bases représentée par la SEQ N° 1, 3 ou 12 ;
(3) un gène qui s'hybride avec un gène de (2) dans des conditions d'hybridation dans lesquelles une hybridation est effectuée dans une solution contenant du SSC 6 x avec 1% de laurylsulfate de sodium, 100 µg/ml d'ADN de sperme de saumon et de la solution de Denhardt 5 x (contenant de la sérumalbumine bovine, de la polyvinylpyrrolidone et du Ficol chacun à une concentration de 0,1 %) à 65°C pendant 20 heures.

2. Gène suivant la revendication 1, qui est obtenu à partir d'une moisissure appartenant au genre *Aspergillus.*

3. Gène suivant la revendication 1 ou 2, codant pour une protéine capable de conférer une résistance à l'auréobasidine.

4. Gène suivant la revendication 3, représenté par la SEQ ID N° 1 dans la Liste des Séquences.

5. ADN antisens d'un gène suivant la revendication 4, représenté par la SEQ ID N° 13 dans la Liste des Séquences.

6. ARN antisens d'un gène suivant la revendication 4, représenté par la SEQ ID N° 14 dans la Liste des Séquences.

7. Plasmide recombinant contenant un gène suivant l'une quelconque des revendications précédentes.

8. Transformant comprenant un plasmide recombinant suivant la revendication 7 introduit dans celui-ci.

9. Protéine régulant la sensibilité à l'auréobasidine codée par un gène suivant la revendication 1 ou 2.

10. Protéine capable de conférer la résistance à l'auréobasidine, dans laquelle au moins l'aminoacide Gly en position 275 de la protéine conférant la sensibilité à l'auréobasidine représentée par la SEQ ID N° 4 dans la Liste des Séquences a été remplacé par un autre aminoacide.

11. Protéine capable de conférer la résistance à l'auréobasidine suivant la revendication 10, dans laquelle l'aminoacide Gly en position 275 a été remplacé par Val.

12. Procédé pour la production de la protéine régulant la sensibilité à l'auréobasidine suivant les revendications 9, 10 ou 11, qui comprend les étapes consistant à mettre en incubation un transformant suivant la revendication 8 et à recueillir la protéine.
